(19) Europäisches Patentamt
European Patent Office
Office européen des brevets

(11) EP 3 321 323 B1

(12) **EUROPEAN PATENT SPECIFICATION**

(45) Date of publication and mention of the grant of the patent:
**17.01.2024 Bulletin 2024/03**

(21) Application number: **16821071.4**

(22) Date of filing: **30.03.2016**

(51) International Patent Classification (IPC):
***C08L 65/00*** *(2006.01)* ***C08K 5/13*** *(2006.01)*
***C08K 5/3435*** *(2006.01)* ***C08K 5/36*** *(2006.01)*
***C08K 5/49*** *(2006.01)* ***G02B 1/04*** *(2006.01)*
***C08J 5/00*** *(2006.01)*

(52) Cooperative Patent Classification (CPC):
(C-Sets available)
**C08K 5/13; C08K 5/3435; C08K 5/36; C08K 5/49; C08L 65/00; G02B 1/04;** C07D 213/74; C08J 5/00

(Cont.)

(86) International application number:
**PCT/JP2016/060578**

(87) International publication number:
**WO 2017/006600 (12.01.2017 Gazette 2017/02)**

(54) **RESIN COMPOSITION, RESIN MOLDED ARTICLE, AND OPTICAL MEMBER**

HARZZUSAMMENSETZUNG, HARZFORMARTIKEL UND OPTISCHES ELEMENT

COMPOSITION DE RÉSINE, ARTICLE MOULÉ EN RÉSINE ET ÉLÉMENT OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2015 JP 2015138103**
**24.09.2015 JP 2015186736**

(43) Date of publication of application:
**16.05.2018 Bulletin 2018/20**

(73) Proprietor: **Zeon Corporation**
**Tokyo 100-8246 (JP)**

(72) Inventors:
- **FURUKO Akira**
  **Tokyo 100-8246 (JP)**
- **SATO Ayumi**
  **Tokyo 100-8246 (JP)**
- **SAWAGUCHI Taichi**
  **Tokyo 100-8246 (JP)**
- **FUJII Kensaku**
  **Tokyo 100-8246 (JP)**
- **HOUKAWA Takashi**
  **Tokyo 100-8246 (JP)**

(74) Representative: **Maiwald GmbH**
**Elisenhof**
**Elisenstraße 3**
**80335 München (DE)**

(56) References cited:
**WO-A1-99/38918**
**WO-A1-2008/047468**
**JP-A- H09 268 250**
**JP-A- 2006 143 931**
**JP-A- 2007 176 960**
**JP-A- 2008 274 135**
**JP-A- 2011 052 154**
**JP-A- 2014 065 756**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08K 5/13, C08L 65/00;**
**C08K 5/3435, C08L 65/00**

## Description

### TECHNICAL FIELD

The present invention relates to a resin composition which is hardly burned during forming, has excellent long-term thermal yellowing resistance (property of being hardly yellowed even when placed at high temperature for a long period) and also suppresses adhesion of foreign matters due to charge, to a resin formed article obtained by forming the resin composition, and to an optical member including the resin formed article.

### BACKGROUND ART

In recent years, cameras and the like have been installed in vehicles in order to grasp surrounding situations and record traveling information and the like for automobiles and the like.

In addition, since the inside of a vehicle such as an automobile becomes extremely hot depending on the seasons, lenses used for such cameras require excellent optical properties such as transparency and furthermore excellent thermal yellowing resistance.

Conventionally, alicyclic-structure-containing polymers have been known as resins excellent in transparency and the like. Further, it is also known that a resin formed article excellent in optical properties can be obtained by combining an alicyclic-structure-containing polymer with various additives such as an antioxidant and a light stabilizer.

For example, Patent Literature 1 describes a specific resin composition prepared by blending a norbornene-based polymer having no polar group, a phosphite ester compound having one phenol structure and a hindered amine compound, and a resin formed article prepared by forming this resin composition. In addition, this literature also describes that the resin formed article has sufficient light stability against short-wavelength laser light such as blue-violet laser. Patent Literatures 2 to 8 further disclose cycloolefin polymer containing resin compositions and molded articles made thereof, comprising different antioxidants.

### CITATION LIST

### PATENT LITERATURE

Patent Literature 1: WO 2012/043721, brochure
Patent Literature 2: JP H09/268259 A
Patent Literature 3: JP 2006/143931 A
Patent Literature 4: JP 2007/176960 A
Patent Literature 5: JP 2014/065756 A
Patent Literature 6: WO 99/38918 A1
Patent Literature 7: WO 2008/047468 A1
Patent Literature 8: JP 2011/052154 A


### SUMMARY OF INVENTION

### TECHNICAL PROBLEM

As described above, a resin formed article excellent in optical properties and useful as a camera lens or the like installed in a vehicle such as an automobile can be obtained by using a resin composition containing an alicyclic-structure-containing polymer and various additives.

However, when the resin composition containing the alicyclic-structure-containing polymer and the various additives was used as a raw material, the resin composition was burned during forming, or a resin formed article excellent in long-term thermal yellowing resistance could not be obtained in some cases.

In addition, even when the resin composition containing an alicyclic-structure-containing polymer and the various additives was used as a raw material, a resin formed article excellent in thermal yellowing resistance could not be obtained in some cases.

The present invention has been made in view of the above circumstances, and the first object of the present invention is to provide a resin composition which is hardly burned during forming, has excellent long-term thermal yellowing resistance and also suppresses adhesion of foreign matters due to charge, a resin formed article obtained by forming the resin composition, and an optical member including the resin formed article.

The second object of the present invention is to provide a resin formed article excellent in optical properties as well as thermal yellowing resistance, and an optical member including the resin formed article.

SOLUTION TO PROBLEM

In order to solve the above problems, the present inventors conducted extensive studies with regard to a resin composition containing an alicyclic-structure-containing polymer, a phenol-based antioxidant and a hindered-amine-based light stabilizer, and a resin formed article obtained by using the resin composition.

As a result, the inventors have obtained a knowledge that the causes for the yellowing of the resin composition and the resin formed article in a heat resistance test include oxidative degradation of resin components and deterioration of additives. As a result of further detailed examination, they have found that (i) the phenol-based antioxidant has functions of preventing burning during forming and preventing yellowing of the resin components under a high temperature condition, while the phenol-based antioxidant itself deteriorates, resulting in yellowing of the resin composition and the resin formed article depending on the conditions, (ii) combination of the phenol-based antioxidant and the hindered amine compound is effective for preventing yellowing of the resin composition and the resin formed article due to deterioration of the phenol-based antioxidant itself, and (iii) on the other hand, when an amount of the added hindered amine compound is increased, the thermal yellowing resistance of the obtained resin composition or resin formed article becomes worse, and furthermore, the resin composition and the resin formed article are readily charged, resulting in a problem of adhesion of foreign matters.

In addition, the present inventors have found that the resin composition prepared by blending at least an alicyclic-structure-containing polymer, a hindered-phenol-based antioxidant optionally having a sulfur atom and/or a phosphorus atom as well as a hindered amine compound in a specific amount respectively, in which a heteroatom-containing compound having a sulfur atom and/or a phosphorus atom (excluding the hindered-phenol-based antioxidant) is blended in a specific amount or less, is excellent in optical properties and suitable as a raw material for producing the resin formed article excellent in thermal yellowing resistance.

Furthermore, the inventors have completed the present invention on the basis of these knowledges.

Thus, one aspect of the invention provides resin compositions of [1] to [3], resin formed articles of [4] and [5], and an optical member of [6], described below.

[1] A resin composition containing an alicyclic-structure-containing polymer, a hindered-phenol-based antioxidant and a hindered amine compound, wherein

the hindered-phenol-based antioxidant is a compound having a group represented by the following formula (1):

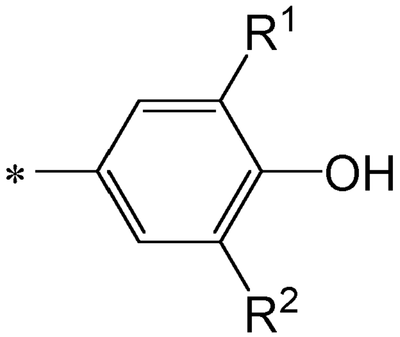

(1)

wherein each of $R^1$ and $R^2$ independently represents a group having 1 or more carbon atoms, and the total number of carbon atoms constituting $R^1$ and $R^2$ is 2 to 20, and * represents a bonding hand,

the hindered amine compound is a compound having a group represented by the following formula (2a):

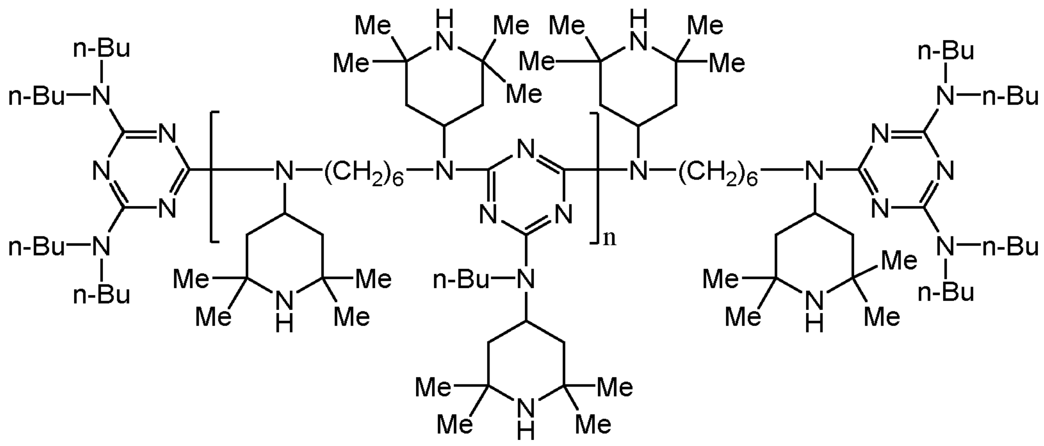

(2a)

wherein n represents any natural number, Me represents a methyl group, and n-Bu represents a n-butyl group, a content of the hindered-phenol-based antioxidant is 0.2 to 2.0 parts by weight based on 100 parts by weight of the alicyclic-structure-containing polymer, and
a content of the hindered amine compound is 0.01 to 0.10 part by weight based on 100 parts by weight of the alicyclic-structure-containing polymer.

[2] The resin composition according to [1], wherein

the content of the hindered-phenol-based antioxidant is 0.2 to 1.0 part by weight based on 100 parts by weight of the alicyclic-structure-containing polymer, and
the content of the hindered amine compound is more than 0.01 to 0.10 part by weight based on 100 parts by weight of the alicyclic-structure-containing polymer.

[3] The resin composition according to [1] or [2], wherein the alicyclic-structure-containing polymer is a norbornene-based polymer.
[4] A resin formed article produced by forming the resin composition according to any of [1] to [3].
[5] The resin formed article according to [4], wherein a yellowness index ($\Delta\delta YI$), measured in a transmission mode using a color difference meter in accordance with JIS K7373, represented by the following equation (I) is 20 or less when a test piece with an optical path length of 3 mm is prepared using the resin formed article as a material and the resulting test piece is allowed to stand under a condition of an oxygen concentration of 21 vol% and a temperature of 125°C for 1,000 hours to carry out a heat resistance test:

$$\Delta\delta YI = \delta YI_1 - \delta YI_0 \qquad (I)$$

wherein $\delta YI_1$ represents a difference between a yellowness index of the test piece after the heat resistance test ($YI_1$) and a yellowness index of a blank (air) ($YI_B$), and $\delta YI_0$ represents a difference between a yellowness index of the test piece before the heat resistance test ($YI_0$) and the yellowness index of the blank (air) ($YI_B$).
[6] An optical member including the resin formed article according to [4] or [5].

ADVANTAGEOUS EFFECTS OF INVENTION

One aspect of the invention provides a resin composition which is hardly burned during forming, has excellent long-term thermal yellowing resistance and also suppresses adhesion of foreign matters due to charge, a resin formed article obtained by forming the resin composition, and an optical member including the resin formed article.
Further, one aspect of the invention provides a resin formed article excellent in optical properties as well as thermal yellowing resistance, and an optical member including the resin formed article.

BRIEF DESCRIPTION OF DRAWINGS

FIG. 1 illustrates a graph summarizing results of the long-term thermal yellowing resistance test (1) in Reference Examples 1 to 9.

FIG. 2 illustrates a graph summarizing the results of the long-term thermal yellowing resistance test (2) in Examples 1 to 9 and Comparative Examples 1 to 6.

DESCRIPTION OF EMBODIMENTS

Hereinafter, the embodiments of the present invention will be classified into 1) a resin composition, and 2) a resin formed article and an optical member, and described in detail.

1) Resin composition

The resin composition according to one embodiment of the invention is characterized in the followings:

(i) the resin composition is a resin composition containing an alicyclic-structure-containing polymer, a hindered-phenol-based antioxidant and a hindered amine compound, wherein
(ii) the hindered-phenol-based antioxidant is a compound having a group represented by the above formula (1),
(iii) the hindered amine compound is a compound having a group represented by the above formula (2a) in its molecule,
(iv) a content of the hindered-phenol-based antioxidant is 0.2 to 2.0 parts by weight based on 100 parts by weight of the alicyclic-structure-containing polymer, and
(v) a content of the hindered amine compound is 0.01 to 0.10 part by weight based on 100 parts by weight of the alicyclic-structure-containing polymer.

(Alicyclic-structure-containing polymer)

The alicyclic-structure-containing polymer constituting the resin composition according to one embodiment of the invention refers to polymers having alicyclic structures in a main chain and/or side chains. Above all, polymers having alicyclic structures in the main chain are preferred because a resin formed article excellent in mechanical strength, heat resistance and the like can be easily obtained.

Examples of the alicyclic structure include a saturated cyclic hydrocarbon (cycloalkane) structure, an unsaturated cyclic hydrocarbon (cycloalkene) structure, and the like. Above all, the cycloalkane structure and the cycloalkene structure are preferred because a resin formed article excellent in mechanical strength, heat resistance and the like can be easily obtained, and the cycloalkane structure is more preferred.

The number of carbon atoms constituting the alicyclic structure is not particularly limited, but is normally a range of 4 to 30, preferably 5 to 20, and more preferably 5 to 15. When the number of carbon atoms constituting the alicyclic structure is within these ranges, a resin formed article having more highly balanced characteristics such as mechanical strength, heat resistance and formability can be easily obtained.

The proportion of repeating units having alicyclic structures in the alicyclic-structure-containing polymer can be appropriately selected depending on the intended purpose. The proportion of the repeating units is normally 30 wt% or more, preferably 50 wt% or more, and more preferably 70 wt% or more based on all repeating units. When the proportion of the repeating units having alicyclic structures in the alicyclic-structure-containing polymer is 30 wt% or more, a resin formed article excellent in heat resistance, transparency and the like can be easily obtained. The remainder other than the repeating units having the alicyclic structures in the alicyclic-structure-containing polymer is not particularly limited, and is appropriately selected depending on the intended purpose.

The weight average molecular weight (Mw) of the alicyclic-structure-containing polymer is not particularly limited, but is normally 5,000 to 500,000, preferably 8,000 to 200,000, and more preferably 10,000 to 100,000. When the weight average molecular weight (Mw) of the alicyclic-structure-containing polymer is within these ranges, the mechanical strength of the resin formed article and the formability in producing the resin formed article can be more highly balanced.

The molecular weight distribution (Mw/Mn) of the alicyclic-structure-containing polymer is not particularly limited, but is normally 1.0 to 4.0, preferably 1.0 to 3.0, and more preferably 1.0 to 2.5.

The weight average molecular weight (Mw) and the number average molecular weight (Mn) of the alicyclic-structure-containing polymer can be determined as standard polyisoprene-equivalent values e.g. by gel permeation chromatography (GPC) using cyclohexane as a solvent.

A glass transition temperature (Tg) of the alicyclic-structure-containing polymer is not particularly limited, but is normally 100 to 200°C, and preferably 130 to 170°C.

When the glass transition temperature (Tg) of the alicyclic-structure-containing polymer is 100°C or higher, a resin formed article excellent in heat resistance can be easily obtained. In addition, the resin composition containing the

alicyclic-structure-containing polymer having a glass transition temperature (Tg) of 200°C or lower shows sufficient flowability during melting and is excellent in formability.

The glass transition temperature (Tg) can be measured in accordance with JIS K 7121.

The alicyclic-structure-containing polymer is preferably an amorphous resin (a resin having no melting point). When the alicyclic-structure-containing polymer is an amorphous resin, a resin formed article more excellent in transparency can be easily obtained.

Specific examples of the alicyclic-structure-containing polymer include (a) a norbornene-based polymer, (b) a monocyclic cycloolefin-based polymer, (c) a cyclic conjugated diene-based polymer, (d) a vinyl alicyclic hydrocarbon-based polymer and the like. Above all, the norbornene-based polymer is preferred because a resin formed article excellent in heat resistance and mechanical strength can be easily obtained.

Note that, these polymers herein refer to not only a polymerization reaction product but also a hydrogenated product thereof.

(a) Norbornene-based polymer

The norbornene-based polymer is a polymer obtained by polymerizing a norbornene-based monomer which is a monomer having a norbornene skeleton, or a hydrogenated product thereof.

Examples of the norbornene-based polymer include a ring-opening polymer of a norbornene-based monomer, a ring-opening polymer of a norbornene-based monomer and another monomer capable of ring-opening copolymerization therewith, hydrogenated products of these ring-opening polymers, an addition polymer of a norbornene-based monomer, an addition polymer of a norbornene-based monomer and another monomer copolymerizable therewith, and the like.

Examples of the norbornene-based monomer include bicyclo[2.2.1]hept-2-ene (trivial name: norbornene) and a derivative thereof (having substituents on the ring), tricyclo[4.3.0$^{1,6}$.1$^{2,5}$]deca-3,7-diene (trivial name: dicyclopentadiene) and a derivative thereof (having substituents on the ring), tetracyclo[9.2.1.0$^{2,10}$.0$^{3,8}$]tetradeca-3,5,7,12-tetraene (methanotetrahydrofluorene: also referred to as 1,4-methano-1,4,4a,9a-tetrahydrofluorene) and a derivative thereof (having substituents on the ring), tetracyclo[4.4.1$^{2,5}$.1$^{7,10}$.0]dodeca-3-ene (trivial name: tetracyclododecene) and a derivative thereof (having substituents on the ring), and the like.

Examples of the substituent include an alkyl group, an alkylene group, a vinyl group, an alkoxycarbonyl group, an alkylidene group and the like.

Examples of the norbornene-based monomer having a substituent include, but are not limited to, 8-methoxycarbonyl-tetracyclo[4.4.0.1$^{2,5}$.1$^{7,10}$]dodeca-3-ene, 8-methyl-8-methoxycarbonyl-tetracyclo[4.4.0.1$^{2,5}$.1$^{7,10}$]dodeca-3-ene, 8-ethylidenetetracyclo[4.4.0.1$^{2,1}$.1$^{7,10}$]dodeca-3-ene and the like.

These norbornene-based monomers may be used either alone or in combination of two or more kinds.

Examples of the other monomers capable of ring-opening copolymerization with the norbornene-based monomer include a monocyclic cycloolefin-based monomer such as cyclohexene, cycloheptene, cyclooctene and derivatives thereof (having substituents on the ring), and the like. Examples of these substituents include the same substituents as those shown as the substituents of the norbornene-based monomer.

Examples of the other monomers capable of addition copolymerization with the norbornene-based monomer include an $\alpha$-olefin having 2 to 20 carbon atoms such as ethylene, propylene, 1-butene, 1-pentene and 1-hexene, and derivatives thereof (having substituents); a cycloolefin such as cyclobutene, cyclopentene, cyclohexene and cyclooctene, and derivatives thereof (having substituents on the ring); a nonconjugated diene such as 1,4-hexadiene, 4-methyl-1,4-hexadiene, 5-methyl-1,4-hexadiene and 1,7-octadiene; and the like. Above all, the $\alpha$-olefin is preferred, and ethylene is particularly preferred. Examples of these substituents include the same substituents as those shown as the substituents of the norbornene-based monomer.

A ring-opening polymer of a norbornene-based monomer, or a ring-opening polymer of a norbornene-based monomer and another monomer capable of ring-opening copolymerization therewith can be synthesized by polymerizing monomer components in the presence of a known ring-opening polymerization catalyst.

Examples of the ring-opening polymerization catalyst include a catalyst including a halide of a metal such as ruthenium and osmium and a nitrate or an acetylacetone compound as well as a reducing agent, or alternatively a catalyst including a halide of a metal such as titanium, zirconium, tungsten and molybdenum or an acetylacetone compound as well as an organoaluminum compound, and the like.

The hydrogenated ring-opening polymer of the norbornene-based monomer can be normally obtained by adding a known hydrogenation catalyst containing a transition metal such as nickel and palladium to a polymerization solution of the above ring-opening polymer to hydrogenate a carbon-carbon unsaturated bond.

An addition polymer of a norbornene-based monomer, or an addition polymer of the norbornene-based monomer and another monomer copolymerizable therewith can be synthesized by polymerizing monomer components in the presence of a known addition polymerization catalyst. Examples of the addition polymerization catalyst include e.g. a catalyst including a titanium, zirconium or vanadium compound and an organoaluminum compound.

Among these norbornene-based polymers, the hydrogenated ring-opening polymer of the norbornene-based monomer is preferable because a resin formed article excellent in heat resistance, mechanical strength and the like can be easily obtained, and as the norbornene-based monomer, the hydrogenated ring-opening polymer of the norbornene-based monomer using tetracyclo[9.2.1.$0^{2,10}$.$0^{3,8}$]tetradeca-3,5,7,12-tetraene is more preferred. The amount of the repeating units derived from tetracyclo[9.2.1.$0^{2,10}0^{3,8}$]tetradeca-3,5,7,12-tetraene in the norbornene-based polymer is preferably 50 wt% or more, and more preferably 70 wt% or more.

(b) Monocyclic cycloolefin-based polymer

Examples of the monocyclic cycloolefin-based polymer include e.g. an addition polymer of a monocyclic cycloolefin monomer such as cyclohexene, cycloheptene and cyclooctene.
The method for synthesizing these addition polymers is not particularly limited, and a known method can be appropriately used.

(c) Cyclic conjugated diene-based polymer

Examples of the cyclic conjugated diene-based polymer include e.g. a polymer prepared by 1,2- or 1,4-addition polymerization of a cyclic conjugated diene-based monomer such as cyclopentadiene, cyclohexadiene, a hydrogenated product thereof, and the like.
The method of synthesizing these addition polymers is not particularly limited, and a known method can be appropriately used.

(d) Vinyl alicyclic hydrocarbon-based polymer

Examples of the vinyl alicyclic hydrocarbon-based polymer include e.g. a polymer of a vinyl alicyclic hydrocarbon-based monomer such as vinyl cyclohexene and vinyl cyclohexane, and hydrogenated products thereof; a hydrogenated product on an aromatic ring moiety in a polymer of a vinyl aromatic monomer such as styrene and $\alpha$-methylstyrene; and the like.
In addition, the vinyl alicyclic hydrocarbon-based polymer may be a copolymer of a vinyl alicyclic hydrocarbon-based monomer or a vinyl aromatic monomer and another monomer copolymerizable with these monomers. Examples of such copolymers include a random copolymer, a block copolymer and the like.
The method for synthesizing these polymers is not particularly limited, and a known method can be appropriately used.

[Hindered-phenol-based antioxidant]

The hindered-phenol-based antioxidant constituting the resin composition according to one embodiment of the invention is a compound having a group represented by the following formula (1), and can capture a peroxy radical (ROO·)

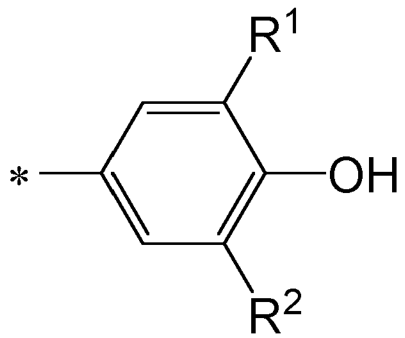

(1)

In the formula (1), each of $R^1$ and $R^2$ independently represents a group having 1 or more carbon atoms, and the total number of carbon atoms constituting $R^1$ and $R^2$ is 2 to 20, preferably 3 to 15, and more preferably 5 to 10. * represents a bonding hand.
Examples of $R^1$ and $R^2$ include an alkyl group such as a methyl group, an ethyl group, a n-propyl group, an isopropyl group, a n-butyl group, an isobutyl group, a s-butyl group and a t-butyl group. In addition, $R^1$ and $R^2$ may be a group in which a sulfur atom is inserted between the carbon-carbon bonds in these alkyl groups.
The number of groups represented by the above formula (1) contained in the hindered-phenol-based antioxidant

is not particularly limited, and it is normally 1 to 10, and preferably 1 to 5.

Although the hindered-phenol-based antioxidant may or may not necessarily have a sulfur atom or a phosphorus atom in its molecule, an antioxidant having neither sulfur atom nor phosphorus atom in its molecule is preferred because a resin formed article more excellent in thermal yellowing resistance can be easily obtained.

Examples of the hindered-phenol-based antioxidant having neither sulfur atom nor phosphorus atom in its molecule include a compound in which the number of the group represented by the above formula (1) is 1, such as 3,5-di-t-butyl-4-hydroxytoluene and octadecyl-3-(3,5-di-t-butyl-4-hydroxyphenyl) propionate; a compound in which the number of the groups represented by the above formula (1) is 2, such as triethylene glycol-bis[3-(3-t-butyl-5-methyl-4-hydroxyphenyl)propionate], triethylene glycol-bis[3-(3,5-di-t-butyl-4-hydroxyphenyl)propionate] and 1,6-hexanediol-bis[3-(3,5-di-t-butyl-4-hydroxyphenyl)propionate]; a compound in which the number of the groups represented by the above formula (1) is 3, such as 1,3,5-trimethyl-2,4,6-tris(3,5-di-t-butyl-4-hydroxybenzyl)benzene; a compound in which the number of the groups represented by the above formula (1) is 4, such as pentaerythritoltetrakis[3-(3,5-di-t-butyl-4-hydroxyphenyl)propionate]; and the like.

Above all, pentaerythritol-tetrakis[3-(3,5-di-t-butyl-4-hydroxyphenyl)propionate] is preferred.

Examples of the hindered-phenol-based antioxidant having a sulfur atom and/or a phosphorus atom in its molecule include 2,2'-thiodiethylbis[3-(3,5-di-t-butyl-4-hydroxyphenyl)propionate, 6-t-butyl-4-[3-(2,4,8,10-tetra-t-butyldibenzo[d,f][1,3,2]dioxaphosphepin-6-yloxy)propyl]-o-cresol, and the like.

The molecular weight of the hindered-phenol-based antioxidant is preferably 200 to 1,500, and more preferably 500 to 1,300. By using the hindered-phenol-based antioxidant having a molecular weight within the above range, a resin composition which is hardly burned during forming and has more excellent long-term thermal yellowing resistance, can be obtained.

[Hindered amine compound]

The hindered amine compound is a compound having a group represented by the following formula (2) in its molecule, and can function as a light stabilizer.

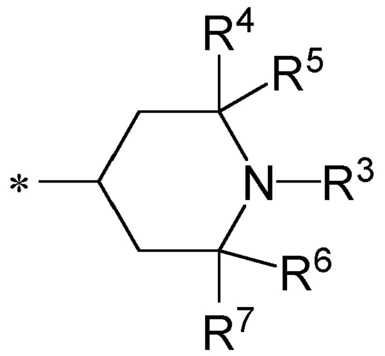

(2)

In the formula (2), $R^3$ represents a hydrogen atom, an alkyl group, a cycloalkyl group, an aralkyl group or an acyl group, and a hydrogen atom is preferred. Each of $R^4$ to $R^7$ independently represents an alkyl group, a cycloalkyl group or an aralkyl group, and the alkyl group is preferred. * represents a bonding hand.

The carbon number in each of the alkyl groups of $R^3$ to $R^7$ is preferably 1 to 15, and more preferably 1 to 10.

Examples of the alkyl groups of $R^3$ to $R^7$ include a methyl group, an ethyl group, a propyl group, a n-butyl group, a s-butyl group, a n-hexyl group, a n-octyl group, a n-decyl group and the like.

The carbon number in each of the cycloalkyl groups of $R^3$ to $R^7$ is preferably 3 to 15, and more preferably 3 to 10. Examples of the cycloalkyl groups of $R^3$ to $R^7$ include a cyclopropyl group, a cyclobutyl group, a cyclopentyl group, a cyclohexyl group, a norbornyl group and the like.

The carbon number in the aralkyl groups of $R^3$ to $R^7$ is preferably 7 to 15, and more preferably 7 to 12.

Examples of the aralkyl groups of $R^3$ to $R^7$ include a benzyl group, a phenethyl group, a naphthylmethyl group and the like.

The carbon number in the acyl groups of $R^3$ to $R^7$ is preferably 2 to 15, and more preferably 2 to 8.

Examples of the acyl groups of $R^3$ to $R^7$ include an acetyl group, a propanoyl group, a butanoyl group, a pentanoyl group, a benzoyl group and the like.

These groups may be groups having substituents such as an alkoxy group and a halogen atom.

The hindered amine compound may be a low-molecular-weight compound or a high-molecular-weight compound.

Examples of the low-molecular-weight hindered amine compound include 1-[2-[3-(3,5-di-t-butyl-4-hydroxyphenyl)propionyloxy]ethyl]-4-[3-3,5-di-t-butyl-4-hydroxyphenyl)propionyloxy]-2,2,6,6-tetramethylpiperidine, bis-(1,2,2,6,6-pentamethyl-4-piperidyl) 2-(3,5-di-t-butyl-4-hydroxybenzyl)-2-n-butylmalonate, N,N',N",N'''-tetrakis-(4,6-bis(butyl-(N-methyl-2,2,6,6-tetramethylpiperidin-4-yl)amino)-triazine-2-yl)-4,7-diazadecane-1,10-amine, bis(2,2,6,6-tetramethyl-4-piperidyl)sebacate, bis(N-methyl-2,2,6,6-tetramethyl-4-piperidyl)sebacate, (2,2,6,6-tetramethyl-4-piperidyl/tridecyl)-1,2,3,4-butanetetracarboxylate, (1,2,2,6,6-pentamethyl-4-piperidyl/tridecyl)-1,2,3,4-butanetetracarboxylate, and the like.

Examples of the high-molecular-weight hindered amine compound include

a polycondensate of dibutylamine, 2,4,6-trichloro-1,3,5-triazine, N,N'-bis(2,2,6,6-tetramethyl-4-piperidyl-1,6-hexamethylenediamine and N-(2,2,6,6-tetramethyl-4-piperidyl)butylamine,
a polycondensate of dibutylamine, 2,4,6-trichloro-1,3,5-triazine, and N,N'-bis(2,2,6,6-tetramethyl-4-piperidyl)butylamine,
poly[{(1,1,3,3-tetramethylbutyl)amino-1,3,5-triazine-2,4-diyl}{(2,2,6,6-tetramethyl-4-piperidyl)imino}hexamethylene { (2,2,6,6-tetramethyl-4-piperidyl)imino}],
a polycondensate of 1,6-hexanediamine-N,N'-bis(2,2,6,6-tetramethyl-4-piperidyl) and morpholine-2,4,6-trichloro-1,3,5-triazine,
poly[(6-morpholino-s-triazine-2,4-diyl)[(2,2,6,6-tetramethyl-4-piperidyl)imino]-hexamethylene[(2,2,6,6-tetramethyl-4-piperidyl)imino],
a polycondensate of dimethyl succinate and 4-hydroxy-2,2,6,6-tetramethyl-1-piperidineethanol,
a mixed esterified product of 1,2,3,4-butanetetracarboxylic acid and 1,2,2,6,6-pentamethyl-4-piperidinol and 3,9-bis(2-hydroxy-1,1-dimethylethyl)-2,4,8,10-tetraoxaspiro[5,5]undecane,
poly[{6-(1,1,3,3-tetramethylbutyl)amino-1,3,5-triazine-2,4-diyl}{(2,2,6,6-tetramethyl-4-piperidyl)imino} hexamethylene{(2,2,6,6-tetramethyl-4-piperidyl)imino}],
a compound represented by the following formula (2a). Above all, only the compound represented by the following formula (2a) is used in the present invention from the viewpoint of obtaining a resin composition which is hardly burned during forming, has excellent long-term thermal yellowing resistance and also suppresses adhesion of foreign matters due to charge.

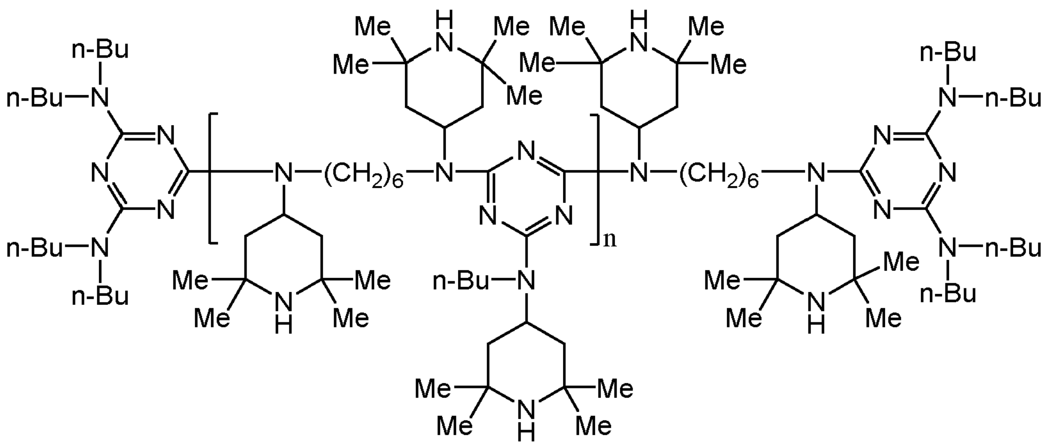

(2a)

In the formula (2a), n represents any natural number, Me represents a methyl group, and n-Bu represents a n-butyl group.

The molecular weight of the hindered amine compound represented by the formula (2a) is preferably 1,000 to 5,000, and more preferably 2,000 to 4,000. By using the hindered amine compound having a molecular weight within the above range, a resin composition which is hardly burned during forming and has excellent long-term thermal yellowing resistance, can be obtained.

The compound represented by the formula (2a) can be synthesized by polycondensation of e.g. dibutylamine, 2,4,6-trichloro-1,3,5-triazine, N,N'-bis(2,2,6,6-tetramethyl-4-piperidyl-1,6-hexamethylenediamine and N-(2,2,6,6-tetramethyl-4-piperidyl)butylamine.

In addition, a commercial product such as CHIMASSORB 2020 FDL (manufactured by BASF SE) can also be used as the compound represented by the formula (2a).

[Resin composition]

The resin composition according to one embodiment of the invention is a resin composition containing an alicyclic-structure-containing polymer, a hindered-phenol-based antioxidant and a hindered amine compound, wherein the hindered-phenol-based antioxidant is a compound having a group represented by the above formula (1), the hindered amine compound is a compound having a group represented by the above formula (2a) in its molecule, a content of the hindered-phenol-based antioxidant is 0.2 to 2.0 parts by weight based on 100 parts by weight of the alicyclic-structure-containing polymer, and a content of the hindered amine compound is 0.01 to 0.10 part by weight based on 100 parts by weight of the alicyclic-structure-containing polymer.
The resin composition according to one embodiment of the invention is preferably either of the following resin composition (A) or resin composition (B) (not claimed).

[Resin composition (A)]

It is a resin composition containing an alicyclic-structure-containing polymer, a hindered-phenol-based antioxidant and a hindered amine compound, wherein the hindered-phenol-based antioxidant is a compound having a group represented by the above formula (1), the hindered amine compound is a compound represented by the above formula (2a), a content of the hindered-phenol-based antioxidant is 0.2 to 1.0 part by weight, preferably 0.1 to 0.8 part by weight, and more preferably 0.1 to 0.6 part by weight based on 100 parts by weight of the alicyclic-structure-containing polymer, and a content of the hindered amine compound is 0.01 to 0.1 part by weight based on 100 parts by weight of the alicyclic-structure-containing polymer.
When the hindered-phenol-based antioxidant or the hindered amine compound to be used is blended in the resin composition, it improves the intended performance, while also causes reduction of other properties. That is, the hindered amine compound is an important additive for improving the thermal yellowing resistance of the resin composition, while the resin composition containing the hindered amine compound is readily charged, and thus it has sometimes caused problems of adhesion of foreign matters. In addition, the phenol-based antioxidant is an important additive for preventing burning during forming, and also has an effect of suppressing yellowing of the resin component under a high temperature condition. On the other hand, when the antioxidant is used in combination with the hindered amine compound, the thermal yellowing resistance of the resin composition tends to rather decrease, as the content of the hindered-phenol-based antioxidant increases. This yellowing is considered to be caused mainly by deterioration of the hindered-phenol-based antioxidant.
In order to solve these problems, the hindered-phenol-based antioxidant and the hindered amine compound are used in combination respectively in an amount within the above range, in the present invention.
That is, in the resin composition according to one embodiment of the invention, the content of the hindered amine compound is set to 0.10 part by weight or less based on 100 parts by weight of the alicyclic-structure-containing polymer, and the content of the hindered-phenol-based antioxidant is set to 0.2 to 1.0 part by weight based on 100 parts by weight of the alicyclic-structure-containing polymer.
The content of the hindered amine compound is set to a small amount, so that the resin composition and the resin formed article can be charged to solve the problem of adhesion of foreign matters. In addition, if the content of the hindered amine compound is small, a phenomenon of decreased thermal yellowing resistance of the resin composition is hardly caused as the hindered-phenol-based antioxidant is added, and the resulting resin composition is hardly burned during forming and has excellent long-term thermal yellowing resistance.
Thus, when the hindered-phenol-based antioxidant and the hindered amine compound in the amounts within the above ranges are used in combination, a resin composition excellent in all of antistatic property, performance of preventing burning during forming and long-term thermal yellowing resistance can be obtained.

[Resin composition (B)] (not claimed)

The resin composition (B) is a resin composition which is prepared by blending at least the alicyclic-structure-containing polymer, the hindered-phenol-based antioxidant optionally having a sulfur atom and/or a phosphorus atom as well as the hindered amine compound and optionally contains a heteroatom-containing compound having a sulfur atom and/or a phosphorus atom (excluding the hindered-phenol-based antioxidant), wherein the blending amount of the hindered-phenol-based antioxidant is 0.1 to 2.0 parts by weight, preferably 0.3 to 1.5 parts by weight, and more preferably 0.4 to 1.0 part by weight based on 100 parts by weight of the alicyclic-structure-containing polymer, the blending amount of the hindered amine compound is 0.005 to 1.0 part by weight, preferably 0.1 to 1.0 part by weight, more preferably 0.2 to 0.8 part by weight, and even more preferably 0.2 to 0.5 part by weight based on 100 parts by weight of the alicyclic-structure-containing polymer, and the blending amount of the heteroatom-containing compound is 0.05 part by weight or less based on 100 parts by weight of the alicyclic-structure-containing polymer.

When the blending amount of the hindered-phenol-based antioxidant is less than 0.1 part by weight or more than 2.0 parts by weight based on 100 parts by weight of the alicyclic-structure-containing polymer, the resulting resin formed article readily becomes poor in thermal yellowing resistance. In addition, when the blending amount of the hindered amine compound is less than 0.005 part by weight or more than 1.0 part by weight based on 100 parts by weight of the alicyclic-structure-containing polymer, the resulting resin formed article readily becomes poor in thermal yellowing resistance.

Although the hindered-phenol-based antioxidant optionally having a sulfur atom and/or a phosphorus atom used for the resin composition (B) may or may not necessarily a sulfur atom or a phosphorus atom in its molecule, an antioxidant having neither sulfur atom nor phosphorus atom in its molecule is preferred because a resin formed article more excellent in thermal yellowing resistance can be easily obtained.

The hindered amine compound used for the resin composition (B) is a compound having the group represented by the above formula (2) in its molecule, and can function as a light stabilizer.

The resin composition (B) is suitable as a raw material for producing a resin formed article excellent in thermal yellowing resistance. Thus, the method of preparing the resin composition, the conditions for forming the resin formed article, and the like may be optimized, so that a resin formed article having a lower yellowness index ($\Delta\delta$YI) can be efficiently obtained.

The resin composition optionally contains polymer compounds other than the alicyclic-structure-containing polymer, and additives other than the hindered-phenol-based antioxidant or the hindered amine compound, as long as the effect of the present invention is not impaired.

However, when the resin composition contains a [heteroatom-containing compound having a sulfur atom and/or a phosphorus atom (excluding the hindered-phenol-based antioxidant)] (hereinafter this compound is referred to as "heteroatom-containing compound" in some cases), the blending amount of the heteroatom-containing compound in preparing the resin composition is 0.05 part by weight or less, preferably 0.02 part by weight or less, and more preferably 0.01 part by weight or less based on 100 parts by weight of the alicyclic-structure-containing polymer. When the blending amount of the heteroatom-containing compound exceeds 0.05 part by weight based on 100 parts by weight of the alicyclic-structure-containing polymer, the resulting resin formed article readily becomes poor in thermal yellowing resistance.

Examples of the polymer compound other than the alicyclic-structure-containing polymer include e.g. an olefin-based soft polymer such as liquid polyethylene, polypropylene, poly-1-butene, ethylene/$\alpha$-olefin copolymer, propylene/$\alpha$-olefin copolymer, ethylene/propylene/diene copolymer (EPDM) and ethylene/propylene/styrene copolymer; an isobutylene-based soft polymer such as polyisobutylene, isobutylene/isoprene rubber and isobutylene/styrene copolymer; a diene-based soft polymer such as polybutadiene, polyisoprene, butadiene/styrene random copolymer, isoprene/styrene random copolymer, acrylonitrile/butadiene copolymer, acrylonitrile/butadiene/styrene copolymer, butadiene/styrene block copolymer, styrene/butadiene/styrene block copolymer, isoprene/styrene copolymer and styrene/isoprene/styrene block copolymer; a silicon-containing soft polymer such as dimethylpolysiloxane, diphenylpolysiloxane and dihydroxypolysiloxane; a soft polymer including an $\alpha,\beta$-unsaturated acid, such as polybutyl acrylate, polybutyl methacrylate, polyhydroxyethyl methacrylate, polyacrylamide, polyacrylonitrile and butyl acrylate/styrene copolymer; a soft polymer including an unsaturated alcohol and an amine or an acyl derivative thereof or an acetal, such as polyvinyl alcohol, polyvinyl acetate, polyvinyl stearate and vinyl acetate/styrene copolymer; an epoxy-based soft polymer such as polyethylene oxide, polypropylene oxide and epichlorohydrin rubber; a fluorine-based soft polymer such as vinylidene fluoride-based rubber and tetrafluoroethylene/propylene rubber; a soft polymer such as natural rubber, polypeptide, protein, polyester-based thermoplastic elastomer, vinyl chloride-based thermoplastic elastomer and polyamide-based thermoplastic elastomer; a terpene phenol resin; and the like.

When the resin composition used in the present invention contains these polymer compounds, the content of these polymer compounds is normally 100 parts by weight or less, and preferably 50 parts by weight or less, based on 100 parts by weight of the alicyclic-structure-containing polymer.

Examples of additives other than the hindered-phenol-based antioxidants or the hindered amine compounds include an antioxidant, a UV absorber, a near-infrared absorber, a plasticizer, an antistatic agent, a release agent and the like.

Examples of the antioxidant include a phosphorus-based antioxidant such as distearyl pentaerythritol diphosphite, bis(2,4-di-t-butylphenyl)pentaerythritol diphosphite, tris(2,4-di-t-butylphenyl)phosphite, tetrakis(2,4-di-t-butylphenyl)4,4'-biphenyl diphosphite, tetrakis(2,4-di-t-butyl-5-methylphenyl)-4,4-biphenylene diphosphonite, trinonylphenyl phosphite and 3,9-bis(2,6-di-t-butyl-4-methylphenoxy)-2,4,8,10-tetraoxa-3,9-diphosphaspiro[5.5]undecane; a sulfur-based antioxidant such as distearyl-3,3'-thiodipropionate, dilauryl-3,3'-thiodipropionate and pentaerythritoltetra(3-laurylthiopropionate); and the like.

Examples of the UV absorber include a benzotriazole-based UV absorber, a benzoate-based UV absorber, a benzophenone-based UV absorber, an acrylate-based UV absorber, a metal complex-based UV absorber, and the like.

Examples of the near-infrared absorber include a cyanine-based near-infrared absorber; a pyrylium-based

infrared absorber; a squarylium-based near-infrared absorber; a croconium-based infrared absorber; an azulenium-based near-infrared absorber; a phthalocyanine-based near-infrared absorber; a dithiol metal complex-based near-infrared absorber; a naphthoquinone-based near-infrared absorber; an anthraquinone-based near-infrared absorber; an indophenol-based near-infrared absorber; azi-based near-infrared absorber, and the like.

Examples of the plasticizer include a phosphate triester-based plasticizer, an aliphatic monobasic acid ester-based plasticizer, a dihydric alcohol ester-based plasticizer, an oxyacid ester-based plasticizer, and the like.

Examples of the antistatic agent include a quaternary ammonium salt, a sulfonate, an alkyl phosphate-based compound, and the like.

Examples of the release agent include fatty acid, fatty acid metal salt, oxy fatty acid, paraffin, low molecular weight polyolefin, fatty acid amide, alkylene bis fatty acid amide, aliphatic ketone, fatty acid-partially saponified ester, fatty acid lower alcohol ester, fatty acid polyhydric alcohol ester, fatty acid polyglycol ester, modified silicone and the like. Above all, a paraffin wax; a fatty acid ester-based release agent such as stearyl stearate, pentaerythritol distearate, pentaerythritol tristearate, pentaerythritol tetrastearate, 1,2-hydroxystearic acid triglyceride and glycerin tristearate; a fatty acid amide-based release agent such as N-stearyl stearic acid amide, N-oleyl stearic acid amide, ethylenebis stearic acid amide, hexamethylenebis behenic acid amide and ethylenebis oleic acid amide; and the like are preferred from the viewpoint of low volatility.

When the resin composition contains these additives, the content thereof can be appropriately decided according to the purpose. The content of these additives is normally 0.1 to 10 parts by weight, preferably 0.2 to 5 parts by weight, and more preferably 0.3 to 2 parts by weight based on 100 parts by weight of the alicyclic-structure-containing polymer.

The method for preparing the resin composition is not particularly limited as long as each additive can be sufficiently dispersed in the alicyclic-structure-containing polymer. For example, the resin composition can be prepared by a method in which each component is dissolved or dispersed in an appropriate solvent and then the solvent is removed (Method $\alpha$), a method in which the alicyclic-structure-containing polymer is heated and melted, to which an additive is added and kneaded (Method $\beta$) or the like.

In the method $\alpha$, the solvent to be used is not particularly limited as long as it can dissolve or disperse each component. Examples of the solvent include aliphatic hydrocarbons such as n-butane, n-pentane, isopentane, n-hexane, n-heptane and isooctane; alicyclic hydrocarbons such as cyclopentane, cyclohexane, methylcyclopentane, methylcyclohexane, decalin, and bicyclo[4.3.0]nonane, tricyclo[4.3.0.$1^{2,5}$]decane; aromatic hydrocarbons such as benzene and toluene; and the like.

In addition, the solution or dispersion obtained after the polymerization reaction or after the hydrogenation reaction may be directly used as the solution or dispersion in the method $\alpha$.

The method for removing the solvent is not particularly limited, and the solvent can be removed by using e.g. a coagulation method, a casting method, a direct drying method, or the like.

In the method $\beta$, kneading can be carried out using e.g. a melt mixer such as a single-screw extruder, a twin-screw extruder, a Banbury mixer, a kneader, a roll and a feeder ruder. The kneading temperature is preferably 200 to 400°C, and more preferably 240 to 300°C. Additionally, when kneading, each component may be added once and kneaded, or may be kneaded in several times while adding the components stepwise.

Among these methods, the method $\alpha$ may be adopted as the resin compositionpreparing method, so that decomposition and deterioration of each component during the preparation process of the resin composition can be suppressed, because a temperature required for the method $\alpha$ is not so high. Hence, the method $\alpha$ is preferred when producing a resin formed article more excellent in thermal yellowing resistance.

In addition, it is preferable to add a hindered-phenol-based antioxidant to the alicyclic-structure-containing polymer at an early stage during the preparation process of the resin composition in order to prevent deterioration of the alicyclic-structure-containing polymer during the preparation process of the resin composition. For example, if the solvent is removed by heating in isolating the alicyclic-structure-containing polymer from the solution or dispersion obtained after the polymerization reaction or after the hydrogenation reaction, it is preferable to add the hindered-phenol-based antioxidant to this solution or dispersion before heating.

In the resin composition according to one embodiment of the invention, the content of the hindered amine compound is small, and thus adhesion of foreign matters by charge is suppressed. Thus, in the optical member obtained by using the resin composition according to one embodiment of the invention, excellent optical performance is maintained for a long period.

Since the resin composition according to one embodiment of the invention sufficiently contains the phenol-based antioxidant, the composition is hardly burned during forming. Consequently, the resin formed article obtained by using the resin composition according to one embodiment of the invention is excellent in transparency and light transmittance.

In the resin composition according to one embodiment of the invention, a small amount of the hindered amine compound and an appropriate amount of the phenol-based antioxidant are used in combination. Thereby, the resin composition according to one embodiment of the invention is excellent in long-term thermal yellowing resistance.

The long-term thermal yellowing resistance of the resin composition according to one embodiment of the invention can be evaluated by e.g. a process that a test piece having an optical path length of 3 mm is prepared using the resin composition according to one embodiment of the invention, and measured for a yellowness index at the optical path length of 3 mm in accordance with JIS K7373 to determine a yellowness index ($\Delta\delta YI$) according to the following formula (I).

$$\Delta\delta YI = \delta YI_1 - \delta YI_0 \qquad (I)$$

In the formula (I), $\delta YI_1$ represents a difference between the yellowness index of the test piece after the heat resistance test ($YI_1$) and the yellowness index of a blank (air) ($YI_B$), and $\delta YI_0$ represents a difference between the yellowness index of the test piece before the heat resistance test ($YI_0$) and the yellowness index of the blank (air) ($YI_B$).

The yellowness index ($\Delta\delta YI$) of the resin formed article according to one embodiment of the invention is normally 20 or lower, and preferably 15 or lower.

Since the resin composition according to one embodiment of the invention has these characteristics, it is suitably used as a forming material for an optical member.

2) Resin formed article and optical member

The resin formed article according to one embodiment of the invention is prepared by forming the resin composition according to one embodiment of the invention.

The resin formed article according to one embodiment of the invention is excellent in thermal yellowing resistance.

The thermal yellowing resistance of the resin formed article can be evaluated by measuring its yellowness index at an optical path length of 3 mm in accordance with JIS K7373 to determine a yellowness index ($\Delta\delta YI$) according to the following formula (I).

$$\Delta\delta YI = \delta YI_1 - \delta YI_0 \qquad (I)$$

In the formula (I), $\delta YI_1$ represents a difference between the yellowness index of the test piece after the heat resistance test ($YI_1$) and the yellowness index of a blank (air) ($YI_B$), and $\delta YI_0$ represents a difference between the yellowness index of the test piece before the heat resistance test ($YI_0$) and the yellowness index of the blank (air) ($YI_B$).

The yellowness index ($\Delta\delta YI$) of the resin formed article according to one embodiment of the invention is normally 20 or lower, and preferably 15 or lower.

Note that, in the heat resistance test, two or more equivalent resin formed articles are prepared, they are classified into a resin formed article for measuring the yellowness index before the heat resistance test ($YI_0$) and a resin formed article for measuring the yellowness index after the heat resistance test ($YI_1$), and for the latter resin formed article for measuring the yellowness index after the heat resistance test ($YI_1$), a heat resistance test may be carried out with the resin formed article as it is before preparation of the test piece, and after the heat resistance test, a test piece having an optical path length of 3 mm may be prepared from the resin formed article as described above after the heat resistance test to measure the yellowness index after the heat resistance test ($YI_1$), where if the yellowness index ($\Delta\delta YI$) is 20 or lower, the resin formed article corresponds to the resin formed article according to one embodiment of the invention.

The resin formed article according to one embodiment of the invention has a portion of 3 mm or longer obtained by forming the resin composition. When a test piece having an optical path length of 3 mm is prepared using this resin formed article as a material and the resulting test piece is subjected to a heat resistance test by allowing it to stand under a condition of an oxygen concentration of 21 vol% and a temperature of 125°C for 1,000 hours, the yellowness index ($\Delta\delta YI$) represented by the above formula (I) is preferably 20 or lower.

As described below, the resin formed article according to one embodiment of the invention is assumed to be used as an optical member or the like, and has a portion of at least 3 mm. Thus, when carrying out the heat resistance test, a test piece having an optical path length of 3 mm can be prepared from the resin formed article, if necessary by using a known resin processing method such as a cutting process.

Note that the "resin formed article having a portion of 3 mm or longer" means "a resin formed article with a shape allowing preparation of a test piece having an optical path length of 3 mm, which is used for a heat resistance test". Examples of the resin formed article having a portion of 3 mm or longer include, but are not limited to, e.g. a resin sheet having a thickness of 3 mm or longer, a lens having a thickness or a diameter of 3 mm or longer, and the like.

The method for forming the resin formed article according to one embodiment of the invention is not particularly

limited, and can be exemplified by an injection forming method, a press forming method, an extrusion blow forming method, an injection blow forming method, a multilayer blow forming method, a connection blow forming method, a double wall blow forming method, a stretch blow forming method, a vacuum forming method, a rotational forming method, and the like. Above all, the injection forming method and the press forming method are preferred because a desired resin formed article can be formed with high dimensional accuracy, and the injection forming method is more preferred.

When forming a resin formed article by the injection forming method, a forming material (the above-described resin composition) is normally put into a hopper of an injection forming machine, plasticized in a high temperature cylinder, and then the molten resin (plasticized resin) is injected from a nozzle into a die. A desired resin formed article can be obtained by cooling and solidifying the molten resin in the die.

The cylinder temperature is appropriately selected in a range of normally 150 to 400°C, preferably 200 to 350°C, and more preferably 250 to 320°C. When the cylinder temperature is excessively low, the flowability of the molten resin is decreased, and sink or distortion may occur in the resin formed article. On the other hand, if the cylinder temperature is excessively high, silver streak may occur due to thermal decomposition of the forming material, or the resin formed article may turn yellowed.

The injection speed at which the molten resin is injected from the cylinder to the die is preferably 1 to 1,000 $cm^3/s$. When the injection speed is within this range, a resin formed article having an excellent appearance shape can be easily obtained.

The injection pressure at which the molten resin is injected from the cylinder to the die is not particularly limited, and may be appropriately set in consideration of the type of the die, flowability of the forming material and the like. The injection pressure is normally 50 to 1,500 MPa.

In the injection forming method, normally, even after the inside of the die is filled with the molten resin, the molten resin in the die is pressurized (hereinafter this pressure is referred to as "preserved pressure") by operating the screw for a certain period until the molten resin in a gate portion of the die is completely cooled and solidified.

Generally, the preserved pressure is set within a range of the die-fastening pressure, and its upper limit is normally 200 MPa or lower, preferably 170 MPa or lower, and more preferably 150 MPa or lower. When the preserved pressure is 200 MPa or lower, a resin formed article with small distortion can be easily obtained.

On the other hand, the lower limit of the preserved pressure is normally 10 MPa or higher, preferably 12 MPa or higher, and more preferably 15 MPa or higher. When the preserved pressure is 10 MPa or higher, the occurrence of the sink is prevented, and a resin formed article excellent in dimensional accuracy can be easily obtained.

The temperature of the die is normally lower than the glass transition temperature (Tg) of the alicyclic-structure-containing polymer in the forming material, preferably a temperature lower than Tg by 0 to 50°C, and more preferably a temperature lower than Tg by 5 to 20°C. When the temperature of the die is within this range, a resin formed article with small distortion can be easily obtained.

Additionally, in the injection forming method, the forming material may be predried, or an inert gas such as nitrogen may be passed from a hopper part of an injection forming machine. The conditions for predrying are not particularly limited, and predrying can be effected e.g. by vacuum drying at 100 to 110°C for 4 to 12 hours.

By performing these treatments, a resin formed article more excellent in transparency can be easily obtained.

The resin formed article according to one embodiment of the invention includes the resin composition according to one embodiment of the invention, which suppresses adhesion of foreign matters due to charge, is hardly burned during forming, and has excellent long-term thermal yellowing resistance.

The resin formed article according to one embodiment of the invention is suitably used as an optical member such as an optical lens, a prism and a light guide. Above all, it is particularly preferably used as an optical member such as a lens used for a camera installed in an automobile.

EXAMPLES

Hereinafter, the present invention will be further described in detail by way of Examples and Comparative Examples. Note that the present invention is not limited to these examples. Hereinafter, the units "parts" and "%" respectively refer to "parts by weight" and "wt%" unless otherwise indicated.

The respective properties were measured in accordance with the following methods.

(1) Weight average molecular weight

In Production Examples, the weight average molecular weight (Mw) of the polymer was measured by gel permeation chromatography (GPC) using cyclohexane as a solvent, and determined as a standard polyisoprene-equivalent value.

As the standard polyisoprene, a standard polyisoprene manufactured by Tosoh Corporation (Mw=602, 1390, 3920, 8050, 13800, 22700, 58800, 71300, 109000, 280000) was used.

The measurement was carried out using three columns manufactured by Tosoh Corporation (TSKgel G5000HXL, TSKgel G4000HXL, and TSKgel G2000HXL) connected in series, under a condition of a flow rate of 1.0 mL/min, an amount of the injected sample of 100 μL and a temperature of the column of 40°C.

(2) Glass transition temperature (Tg)

The glass transition temperature was measured using a differential scanning calorimeter (DSC6220SII, manufactured by SII NanoTechnology Inc.) at an increase rate of 10°C/min in accordance with JIS K6911.

(3) Thermal yellowing resistance test

A thermal yellowing resistance test was carried out using the resin formed article obtained in Examples or Comparative Examples as a test piece in accordance with the following method.

[Thermal yellowing resistance test (1)]

The yellowness index (YI) of the test piece was measured in a transmission mode using a color difference meter (product name "SE-2000", manufactured by NIPPON DENSHOKU INDUSTRIES Co.,LTD) in accordance with JIS K7373. Note that, at this time, yellowness index of only air was measured as a blank.

Subsequently, the test piece was allowed to stand under a condition of an oxygen concentration of 21 vol% and a temperature of 125°C for 1,000 hours and subjected to a heat resistance test, and then its yellowness index was measured in the same manner as described above.

Subsequently, yellowness index (ΔδYI) was determined on the bases of the above formula (I). That means, the smaller the ΔδYI is, the lower the yellowing at a high temperature is, with better heat resistance.

[Thermal yellowing resistance test (2)]

The test was carried out in the same manner as the thermal yellowing resistance test (1) except that the heating condition for the test piece was changed with an oxygen concentration of 21 vol%, a temperature of 135°C and a duration of 480 hours.

(4) Initial oxidation temperature

The resin compositions obtained in Examples or Comparative Examples were subjected to thermogravimetry to measure the initial oxidation temperature. On the basis of the measured results, the effect of suppressing burning during forming was evaluated in accordance with the following criteria.

Good: The initial oxidation temperature is 240°C or higher.
Bad: The initial oxidation temperature is lower than 240°C.

[Production Example 1] Production of alicyclic-structure-containing polymer (I)

To a polymerization reactor whose inside had been dried and replaced by nitrogen, 2.0 parts (1% based on the total amount of monomers used for polymerization) of monomer mixture consisting of 10% of dicyclopentadiene, 75% of tetracyclododecene and 15% of methanotetrahydrofluorene, 785 parts of dehydrated cyclohexane, 1.21 parts of a molecular weight modifier (1-hexene), 0.98 part of a n-hexane solution of diethylaluminum ethoxide (concentration: 19%), and 11.7 parts of a toluene solution of tungsten (phenylimide) tetrachloride-tetrahydrofuran (concentration: 2.0%) were put, and stirred at 50°C for 10 minutes.

Subsequently, 198.0 parts of the monomer mixture having the same composition as one described above was continuously dropped into the polymerization reactor while stirring the whole content maintained at 50°C for 150 minutes. After completion of the drop, stirring was continued for 30 minutes, then 4 parts of isopropyl alcohol was added to terminate the polymerization reaction. As a result of measuring the polymerization solution by gas chromatography, the conversion ratio of the monomer into the polymer was 100%.

The polymer in the resulting polymerization solution had a number average molecular weight (Mn) of 14,000, a weight average molecular weight (Mw) of 24,000, and a molecular weight distribution (Mw/Mn) of 1.7.

To 240 parts of this polymerization solution, 4 parts of diatomaceous earthsupported nickel catalyst ("T8400RL", nickel carrying ratio: 58%, manufactured by JGC Catalysts and Chemicals Ltd.) was added, and subjected to hydrogenation reaction in an autoclave at 4.4 MPa and 190°C for 5 hours. After the hydrogenation reaction, the catalyst residue

in the hydrogenation solution was filtered off to obtain a colorless transparent solution (hydrogenation reaction solution I). The hydrogenation ratio in the hydrogenation reaction was 99% or higher.

While stirring a mixed solution of 250 parts of acetone and 250 parts of isopropanol, the hydrogenation solution I was poured into this mixed solution to precipitate a hydrogenated polymer, which was taken by filtration. The resulting hydrogenated polymer was washed with 200 parts of acetone and then dried in a vacuum dryer at 100°C depressurized to 0.13 kPa or lower for 24 hours.

The resulting hydrogenated polymer [alicyclic-structure-containing polymer (I)] had a number average molecular weight (Mn) of 13,700, a weight average molecular weight (Mw) of 23,300, a molecular weight distribution (Mw/Mn) of 1.7, and a glass transition temperature (Tg) of 152°C.

[Production Example 2] Production of alicyclic-structure-containing polymer (II)

To a polymerization reactor whose inside had been dried and replaced by nitrogen, 960 parts of toluene, 220 parts of tetracyclododecene and 0.166 part of 1-hexene were charged, and a temperature of the solvent was raised to 40°C while stirring at a rotation of 300 to 350 rpm.

On the other hand, 23.5 parts of toluene, 0.044 part of rac-ethylenebis (1-indenyl) zirconium dichloride and 6.22 parts of a toluene solution of 9.0% methylaluminoxane (TMAO-200 series, manufactured by Tosoh Finechem Corporation) were mixed in a glass container to obtain a catalyst solution.

When the temperature of the solvent in the reactor reached 40°C, the catalyst solution was added to the reactor, and immediately thereafter ethylene gas at 0.08 MPa was introduced into the liquid phase to initiate polymerization. In relation to a position of a spouting port for ethylene, (B)/(A) which is a ratio of the distance (B) between the ethylene spouting port and the liquid level to the distance (A) between the bottom of the reactor and the liquid level, was 0.60. When ethylene gas was consumed, ethylene gas was automatically supplied, so that the pressure of ethylene gas was kept constant. After 30 minutes, introduction of ethylene gas was terminated, the reactor was depressurized, and then 5 parts of methanol was added to terminate the polymerization reaction.

The resulting reaction solution was filtered with RADIOLITE #800, and poured into isopropanol containing 0.05% of hydrochloric acid to precipitate a polymer. The precipitated polymer was separated, washed, and dried under reduced pressure at 100°C for 15 hours.

The resulting polymer [alicyclic-structure-containing polymer (II)] had a number average molecular weight (Mn) of 22,000, a weight average molecular weight (Mw) of 56,000, a molecular weight distribution (Mw/Mn) of 2.6, and a glass transition temperature (Tg) of 145°C.

[Reference Example 1]

100 parts of the alicyclic-structure-containing polymer (I) obtained in Production Example 1 and 0.20 part of pentaerythritol-tetrakis[3-(3,5-di-t-butyl-4-hydroxyphenyl)propionate] (product name: "IRGANOX (registered trademark) 1010" manufactured by BASF SE) (hereinafter referred to as "hindered-phenol-based antioxidant (1)" or "H.P." in some cases) as the hindered-phenol-based antioxidant were kneaded with a twin-screw mixer and extruded to obtain a pelletized resin composition 1.

The pellet was dried by heating at 80°C for 4 hours and then put into an injection forming machine (ROBOSHOT $\alpha$-100B, manufactured by FANUC CORPORATION) and injection-formed with a cylinder temperature of 280°C to obtain a flat plate-shaped resin formed article 1 of 65 mm $\times$ 65 mm $\times$ 3 mm.

A long-term thermal yellowing resistance test (1) was carried out using the resulting resin formed article 1 as a test piece. The results are shown in Table 1.

[Reference Examples 2 and 3]

Resin compositions 2 and 3 were prepared in the same manner as Reference Example 1 except that the content of the hindered-phenol-based antioxidant (1) was changed to the amount shown in Table 1, and the compositions were used to obtain resin formed articles 2 and 3, which were subjected to the long-term thermal yellowing resistance test (1). The results are shown in Table 1.

[Reference Example 4]

100 parts of the hydrogenated polymer obtained in Production Example 1, 0.20 part of hindered-phenol-based antioxidant (1) as a hindered-phenol-based antioxidant, and 0.30 part of polycondensate of dibutylamine, 2,4,6-trichloro-1,3,5-triazine, N,N'-bis(2,2,6,6-tetramethyl-4-piperidyl-1,6-hexamethylenediamine, N-(2,2,6,6-tetramethyl-4-piperidyl)butylamine (product name: "CHIMASSORB (registered trademark) 2020 FDL" manufactured by BASF SE) (hereinafter referred to as "hindered amine compound (1)" or "H.A." in some cases) as the hindered amine compound were kneaded with a twin-screw mixer and extruded to prepare a pelletized resin composition 4, and the composition was used to obtain a resin formed article 4, which was subjected to the long-term thermal yellowing resistance test (1). The results are shown in Table 1.

[Reference Examples 5 to 9]

Resin compositions 5 to 9 were prepared in the same manner as Reference Example 4 except that the contents of the hindered-phenol-based antioxidant (1) and the hindered amine compound (1) were changed to the contents shown in Table 1, and the compositions were used to obtain resin formed articles 5 to 9, which were subjected to the long-term thermal yellowing resistance test (1). The results are shown in Table 1. In addition, a graph summarizing the results of the long-term thermal yellowing resistance test (1) of Reference Examples 1 to 9 is shown in Figure 1.

Table 1

| | | Reference Example | | | | | | | | |
|---|---|---|---|---|---|---|---|---|---|---|
| | | 1 | 2 | 3 | 4 | 5 | 6 | 7 | 8 | 9 |
| Resin composition | No. | 1 | 2 | 3 | 4 | 5 | 6 | 7 | 8 | 9 |
| | Alicyclic- structure-containing polymer (I) (parts) | 100 | 100 | 100 | 100 | 100 | 100 | 100 | 100 | 100 |
| | H.P. (parts) | 0.20 | 0.45 | 0.80 | 0.20 | 0.45 | 0.80 | 0.20 | 0.45 | 0.80 |
| | H.A. (parts) | 0.00 | 0.00 | 0.00 | 0.30 | 0.30 | 0.30 | 0.50 | 0.50 | 0.50 |
| Long-term thermal yellowing resistance test (1) ($\Delta\delta$YI) | | 79 | 26 | 24 | 15 | 25 | 36 | 18 | 29 | 40 |

The followings can be seen from Table 1 and Figure 1.

In the resin compositions 1 to 3 (Reference Examples 1 to 3) containing no hindered amine compound (1), the $\Delta\delta$YI decreases as the content of the hindered-phenol-based antioxidant (1) increases.

On the other hand, in the resin compositions 4 to 9 (Reference Examples 4 to 9) containing the hindered amine compound (1), the $\Delta\delta$YI increases as the content of the hindered-phenol-based antioxidant (1) increases.

As described above, it is understood that the influence of the hindered-phenol-based antioxidant on the yellowing property of the resin composition greatly differs depending on the difference in the presence or absence of the hindered amine compound.

From these facts, it is considered that in the resin composition containing no hindered amine compound, the resin is more hardly deteriorated as the content of the hindered-phenol-based antioxidant increases, and the yellowing is further suppressed. On the other hand, in the resin composition containing the hindered amine compound, a deteriorated product of the hindered-phenol-based antioxidant also increases as the content of the hindered-phenol-based antioxidant increases, and it is considered that the test piece thereby turns yellowed.

[Examples 1 to 9] (Examples 1, 4 and 7 are not claimed)

Resin compositions 10 to 18 were prepared in the same manner as Reference Example 4 except that the contents of the hindered-phenol-based antioxidant (1) and the hindered amine compound (1) were changed to the contents described in Table 2, and the compositions were used to obtain resin formed articles 10 to 18, which were subjected to the long-term thermal yellowing resistance test (2) and measurement of the initial oxidation temperature. The results are shown in Table 2.

[Examples 10 and 11]

Resin compositions 19 and 20 were prepared in the same manner as Reference Example 4 except that the alicyclic-structure-containing polymer (II) was used instead of the alicyclic-structure-containing polymer (I) and that the contents of the hindered-phenol-based antioxidant (1) and the hindered amine compound (1) were changed to the contents described in Table 2, and the compositions were used to obtain resin formed articles 19 and 20, which were subjected to the long-term thermal yellowing resistance test (2) and measurement of the initial oxidation temperature.

The results are shown in Table 2.

[Comparative Examples 1 to 6]

Resin compositions 21 to 26 were prepared in the same manner as Reference Example 4 except that the contents of the hindered-phenol-based antioxidant (1) and the hindered amine compound (1) were changed to the contents described in Table 2, and the compositions were used to obtain resin formed articles 21 to 26, which were subjected to the long-term thermal yellowing resistance test (2) and measurement of the initial oxidation temperature. The results are shown in Table 2. In addition, a graph summarizing the results of the long-term thermal yellowing resistance test (2) of Examples 1 to 9 and Comparative Examples 1 to 6 is shown in Figure 2. Examples 1, 4 and 7 are not claimed.

Table 2

| | | Example | | | | | | | | | | | Comparative Example | | | | | |
|---|---|---|---|---|---|---|---|---|---|---|---|---|---|---|---|---|---|---|
| | | 1 | 2 | 3 | 4 | 5 | 6 | 7 | 8 | 9 | 10 | 11 | 1 | 2 | 3 | 4 | 5 | 6 |
| Resin composition | No. | 10 | 11 | 12 | 13 | 14 | 15 | 16 | 17 | 18 | 19 | 20 | 21 | 22 | 23 | 24 | 25 | 26 |
| | Alicyclic-structure-containing polymer (1) (parts) | 100 | 100 | 100 | 100 | 100 | 100 | 100 | 100 | 100 | - | - | 100 | 100 | 100 | 100 | 100 | 100 |
| | Alicyclic-structure-containing polymer (II) (parts) | - | - | - | - | - | - | - | - | - | 100 | 100 | - | - | - | - | - | - |
| | H.P. (parts) | 0.10 | 0.20 | 0.35 | 0.10 | 0.20 | 0.35 | 0.10 | 0.20 | 0.35 | 0.20 | 0.40 | 0.10 | 0.20 | 0.35 | 0.05 | 0.05 | 0.05 |
| | H.A. (parts) | 0.02 | 0.02 | 0.02 | 0.06 | 0.06 | 0.06 | 0.10 | 0.10 | 0.10 | 0.10 | 0.10 | - | - | - | 0.02 | 0.06 | 0.10 |
| Long-term thermal yellowing resistance test (2) ($\Delta\delta YI$) | | 13 | 13 | 12 | 9 | 9 | 11 | 10 | 10 | 11 | 12 | 15 | 108 | 62 | 31 | 14 | 11 | 10 |
| Initial oxidation temperature | | Good | Good | Good | Good | Good | Good | Good | Good | Good | Good | Good | Good | Good | Good | Bad | Bad | Bad |

From Table 2 and Figure 2, the followings can be seen.

The resin compositions 10 to 20 in Examples 1 to 11 are excellent in long-term thermal yellowing resistance. This long-term thermal yellowing resistance has been achieved while sufficiently containing the hindered-phenol-based antioxidant (1). Thus, the effect of the hindered-phenol-based antioxidant (1) prevents these resin compositions from burning during forming.

Additionally, in these resin compositions, the content of the hindered amine compound (1) is small, and thus they are difficult to charge and hardly cause problems of adhesion of foreign matters.

On the other hand, since the resin compositions 21 to 23 of Comparative Examples 1 to 3 do not contain the hindered amine compound (1), they are poor in the long-term thermal yellowing resistance.

In addition, although the resin compositions 24 to 26 of Comparative Examples 4 to 6 are excellent in the long-term thermal yellowing resistance, the contents of the hindered-phenol-based antioxidant (1) is small. For this reason, these resin compositions tend to have a problem of burning during forming because of low initial oxidation temperature.

[Production Example 3]

To a polymerization reactor whose inside had been replaced by nitrogen, 690 parts of dehydrated toluene, 210 parts of tetracyclo[9.2.1.$0^{2,10}$.$0^{3,8}$]tetradeca-3,5,7,12-tetraene, 75 parts of tetracyclo[4.4.$1^{2,5}$.$1^{7,10}$.0]dodeca-3-ene, 15 parts of bicyclo[2.2.1]hept-2-ene, 1.1 parts of 1-hexene, 11 parts of a 0.3% toluene solution of tungsten chloride and 0.5 part of triisobutylaluminum were put, and subjected to ring-opening polymerization reaction at 1 atom and 60°C for 1 hour.

The polymer in the resulting polymerization solution had a number average molecular weight (Mn) of 14,000, a weight average molecular weight (Mw) of 24,000, and a molecular weight distribution (Mw/Mn) of 1.7.

To 240 parts of this polymerization solution, 4 parts of diatomaceous earthsupported nickel catalyst ("T8400RL", nickel carrying ratio: 58%, manufactured by JGC Catalysts and Chemicals Ltd.) was added, and subjected to hydrogenation reaction in an autoclave at 45 kgf/$cm^2$ and 190°C for 5 hours. After the hydrogenation reaction, the catalyst residue in the hydrogenation reaction solution was filtered off to obtain a colorless transparent solution (hydrogenation reaction solution III). The hydrogenation ratio in the hydrogenation reaction was 99% or higher.

The resulting hydrogenated polymer had a number average molecular weight (Mn) of 16,500, a weight average molecular weight (Mw) of 28,000, a molecular weight distribution (Mw/Mn) of 1.7, and a glass transition temperature (Tg) of 145°C.

(Examples 12 to 23 are not claimed)

[Example 12]

To the hydrogenation solution III obtained in Production Example 3, 0.8 part of pentaerythritol-tetrakis[3-(3,5-di-t-butyl-4-hydroxyphenyl)propionate] ("IRGANOX (registered trademark) 1010" manufactured by BASF SE) (hereinafter referred to as "hindered-phenol-based antioxidant (a1)") as the hindered-phenol-based antioxidant and 0.3 part of polycondensate of dibutylamine, 2,4,6-trichloro-1,3,5-triazine, N,N'-bis(2,2,6,6-tetramethyl-4-piperidyl-1,6-hexamethylenediamine, N-(2,2,6,6-tetramethyl-4-piperidyl)butylamine ("CHIMASSORB (registered trademark) 2020 FDL" manufactured by BASF SE) (hereinafter referred to as "hindered amine compound (b1)") as the hindered amine compound were added based on 100 parts of the hydrogenated polymer, and then foreign matters were removed by filtration using a filter ("ZETAPLUS (registered trademark) 30H", pore diameter: 0.5 to 1 μm, manufactured by CUNO Filter Systems) and a metallic fiber filter (pore diameter: 0.4 μm, manufactured by NICHIDAI CO.,LTD.).

Subsequently, the resulting filtrate was put into a cylindrical concentration dryer (manufactured by Hitachi, Ltd.), from which cyclohexane as a solvent and other volatile components were removed under a condition of a temperature of 290°C and a pressure of 1 kPa or lower, extruded into a strand form in a molten state from a die directly connected to a concentrator, cooled with water, and then cut with a pelletizer ("OSP-2", manufactured by OSADA SEISAKUSHO) to obtain a pelletized resin composition 27.

The pellet was dried by heating at 80°C for 4 hours and then put into an injection forming machine ("ROBOSHOT α-100B", manufactured by FANUC CORPORATION) and injection-formed with a cylinder temperature of 280°C to obtain a flat plate-shaped resin formed article 27 of 65 mm × 65 mm × 3 mm.

A thermal yellowing resistance test was carried out using the resin formed article 27 as a test piece. The results are shown in Table 3.

[Example 13]

A resin composition 28 and a resin formed article 28 were obtained in the same manner as Example 12 except that 0.8 part of 6-t-butyl-4-[3-(2,4,8,10-tetra-t-butyldibenzo[d,f][1,3,2]dioxaphosphepin-6-yloxy)propyl]-o-cresol ("SUMILIZER GP", manufactured by Sumitomo Chemical Co., Ltd.) (hereinafter referred to as "hindered-phenol-based antioxidant (a2)") was added instead of the hindered-phenol-based antioxidant (a1) in Example 12.

A thermal yellowing resistance test was carried out using the resulting resin formed article 28 as a test piece. The results are shown in Table 3.

[Example 14]

A resin composition 29 and a resin formed article 29 were obtained in the same manner as Example 12 except that 0.3 part of poly[{ 6-(1,1,3,3-tetramethylbutyl)amino-1,3,5-triazine-2,4-diyl}{(2,2,6,6-tetramethyl-4-piperidyl)imino}hexamethylene{(2,2,6,6-tetramethyl-4-piperidyl)imino}] ("Chimassorb (registered trademark) 944FDL", manufactured by BASF SE) (hereinafter referred to as " hindered amine compound (b2)") was added instead of the hindered amine compound (b1) in Example 12.

A thermal yellowing resistance test was carried out using the resulting resin formed article 29 as a test piece. The results are shown in Table 3.

[Example 15]

A resin composition 30 and a resin formed article 30 were obtained in the same manner as Example 12 except that the amount of the added hindered-phenol-based antioxidant (a1) was changed to 0.3 part based on 100 parts of the hydrogenated polymer in Example 12.

A thermal yellowing resistance test was carried out using the resulting resin formed article 30 as a test piece. The results are shown in Table 3.

[Example 16]

A resin composition 31 and a resin formed article 31 were obtained in the same manner as Example 12 except that the amount of the added hindered-phenol-based antioxidant (a1) was changed to 2.0 parts based on 100 parts of the hydrogenated polymer in Example 12.

A thermal yellowing resistance test was carried out using the resulting resin formed article 31 as a test piece. The results are shown in Table 3.

[Example 17]

A resin composition 32 and a resin formed article 32 were obtained in the same manner as Example 12 except that the amount of the added hindered amine compound (b 1) was changed to 1.0 part based on 100 parts of the hydrogenated polymer in Example 12.

A thermal yellowing resistance test was carried out using the resulting resin formed article 32 as a test piece. The results are shown in Table 3.

[Example 18]

A resin composition 33 and a resin formed article 33 were obtained in the same manner as Example 12 except that when preparing the resin composition, 0.05 part of 3,9-bis(2,6-di-t-butyl-4-methylphenoxy)-2,4,8,10-tetraoxa-3,9-diphosphaspiro[5.5]undecane ("PEP-36", manufactured by ADEKA CORPORATION) (hereinafter referred to as "heteroatom-containing compound (c1)") as a phosphoruscontaining antioxidant was further added based on 100 parts of the hydrogenated polymer in Example 12.

A thermal yellowing resistance test was carried out using the resulting resin formed article 33 as a test piece. The results are shown in Table 3.

[Example 19]

A resin composition 34 and a resin formed article 34 were obtained in the same manner as Example 12 except that when preparing the resin composition, 0.05 part of pentaerythritol-tetrakis(3-laurylthiopropionate) ("SUMILIZER-TP-D", manufactured by Sumitomo Chemical Co.,Ltd.) (hereinafter referred to as "heteroatom-containing compound (c2)") as a sulfur-containing antioxidant was further added based on 100 parts of the hydrogenated polymer in Example 12.

A thermal yellowing resistance test was carried out using the resulting resin formed article 34 as a test piece. The results are shown in Table 3.

[Example 20]

A resin composition 35 and a resin formed article 35 were obtained in the same manner as Example 12 except that the amount of the added hindered-phenol-based antioxidant (a1) was changed to 0.1 part based on 100 parts of the hydrogenated polymer and the amount of the added hindered amine compound (b1) was changed to 0.1 part based on 100 parts of the hydrogenated polymer in Example 12.
A thermal yellowing resistance test was carried out using the resulting resin formed article 35 as a test piece. The results are shown in Table 3.

[Example 21]

A resin composition 36 and a resin formed article 36 were obtained in the same manner as Example 12 except that the amount of the added hindered-phenol-based antioxidant (a1) was changed to 2.0 parts based on 100 parts of the hydrogenated polymer and the amount of the added hindered amine compound (b1) was changed to 1.0 part based on 100 parts of the hydrogenated polymer in Example 12.
A thermal yellowing resistance test was carried out using the resulting resin formed article 36 as a test piece. The results are shown in Table 3.

[Example 22]

While stirring a mixed solution of 250 parts of acetone and 250 parts of isopropanol, the hydrogenation reaction solution III obtained in Production Example 3 was poured into this mixed solution to precipitate a hydrogenated polymer, which was taken by filtration. The resulting hydrogenated polymer was washed with 200 parts of acetone and then dried in a vacuum dryer at 100°C depressurized to 1 mmHg or lower for 24 hours.
100 parts of the resulting hydrogenated polymer, 0.8 part of hindered-phenol-based antioxidant (a1) and 0.3 part of hindered amine compound (b1) were kneaded in a twin-screw kneader and extruded to obtain a pelletized resin composition 37.
Subsequently, a resin formed article 37 was obtained in the same manner as Example 12 except that the resin composition 37 was used instead of the resin composition 12 in Example 12.
A thermal yellowing resistance test was carried out using the resulting resin formed article 37 as a test piece. The results are shown in Table 3.

[Example 23]

A resin composition 38 and a resin formed article 38 were obtained in the same manner as Example 12 except that the amount of the added hindered-phenol-based antioxidant (a1) was changed to 0.1 part based on 100 parts of the hydrogenated polymer and the amount of the added hindered amine compound (b1) was changed to 0.005 part based on 100 parts of the hydrogenated polymer in Example 12.
A thermal yellowing resistance test was carried out using the resulting resin formed article 38 as a test piece. The results are shown in Table 3.

[Comparative Example 7]

A resin composition 39 and a resin formed article 39 were obtained in the same manner as Example 12 except that when preparing the resin composition, 0.1 part of heteroatom-containing compound (c1) was further added based on 100 parts of the hydrogenated polymer in Example 12.
A thermal yellowing resistance test was carried out using the resulting resin formed article 39 as a test piece. The results are shown in Table 3.

[Comparative Example 8]

A resin composition 40 and a resin formed article 40 were obtained in the same manner as Example 12 except that when preparing the resin composition, 0.1 part of heteroatom-containing compound (c2) was further added based on 100 parts of the hydrogenated polymer in Example 12.
A thermal yellowing resistance test was carried out using the resulting resin formed article 40 as a test piece. The results are shown in Table 3.

[Comparative Example 9]

A resin composition 41 and a resin formed article 41 were obtained in the same manner as Example 12 except that the hindered amine compound (b1) was not added in Example 12.
A thermal yellowing resistance test was carried out using the resulting resin formed article 41 as a test piece. The results are shown in Table 3.

[Comparative Example 10]

A resin composition 42 and a resin formed article 42 were obtained in the same manner as Example 12 except that the amount of the added hindered amine compound (b1) was changed to 2.0 parts based on 100 parts of the hydrogenated polymer in Example 12.
A thermal yellowing resistance test was carried out using the resulting resin formed article 42 as a test piece. The results are shown in Table 3.

[Comparative Example 11]

A resin composition 43 and a resin formed article 43 were obtained in the same manner as Example 12 except that the amount of the added hindered-phenol-based antioxidant (a1) was changed to 0.05 part based on 100 parts of the hydrogenated polymer and the amount of the added hindered amine compound (b1) was changed to 0.5 part based on 100 parts of the hydrogenated polymer in Example 12.
A thermal yellowing resistance test was carried out using the resulting resin formed article 43 as a test piece. The results are shown in Table 3.

[Comparative Example 12]

A resin composition 44 and a resin formed article 44 were obtained in the same manner as Example 12 except that the amount of the added hindered-phenol-based antioxidant (a1) was changed to 3.0 parts based on 100 parts of the hydrogenated polymer in Example 12.
A thermal yellowing resistance test was carried out using the resulting resin formed article 44 as a test piece. The results are shown in Table 3.

[Comparative Example 13]

A resin composition 45 and a resin formed article 45 were obtained in the same manner as Example 15 except that the temperature of the cylinder during injection forming was changed to 320°C in Example 15.
A thermal yellowing resistance test was carried out using the resulting resin formed article 45 as a test piece. The results are shown in Table 3.

[Comparative Example 14]

A resin composition 46 and a resin formed article 46 were obtained in the same manner as Example 22 except that the amount of the added hindered-phenol-based antioxidant (a1) was changed to 0.1 part based on 100 parts of the hydrogenated polymer and the amount of the added hindered amine compound (b1) was changed to 0.1 part based on 100 parts of the hydrogenated polymer in Example 22.
A thermal yellowing resistance test was carried out using the resulting resin formed article 46 as a test piece. The results are shown in Table 3.

[Comparative Example 15]

A resin composition 47 and a resin formed article 47 were obtained in the same manner as Example 15 except that the amount of the added hindered amine compound (b 1) was changed to 0.002 part based on 100 parts of the hydrogenated polymer in Example 15.
A thermal yellowing resistance test was carried out using the resulting resin formed article 47 as a test piece. The results are shown in Table 3.

[Comparative Example 16]

A resin composition 48 and a resin formed article 48 were obtained in the same manner as Example 12 except that the amount of the added hindered amine compound (b1) was changed to 0.002 part based on 100 parts of the hydrogenated polymer in Example 12.

A thermal yellowing resistance test was carried out using the resulting resin formed article 48 as a test piece. The results are shown in Table 3. Examples 12 to 23 are not claimed.

Table 3

| | Resin composition | | | | | | | | Forming condition | Thermal yellowing resistance test |
|---|---|---|---|---|---|---|---|---|---|---|
| | Content (parts) | | | | | | | Method for adding additives | Cylinder temperature [°C] | $\Delta\delta$YI |
| | | Hindered-phenol-based anti-oxidait | | Hindered amine compound | | Heteroatom-cortaining compound | | | | |
| | Alicyclic- structure-containing polymer (Production Example 1) | (a1) | (a2) | (b1) | (b2) | (c1) | (c2) | | | |

(continued)

| | | Resin composition | | | | | | | | Forming condition | Thermal yellowing resistance test |
|---|---|---|---|---|---|---|---|---|---|---|---|
| | | Content (parts) | | | | | | | Method for adding additives | Cylinder temperature [°C] | ΔδYI |
| | | | Hindered-phenol-based anti-oxidait | | Hindered amine compound | | Heteroatom-cortaining compound | | | | |
| | | Alicyclic- structure-containing polymer (Production Example 1) | (a1) | (a2) | (b1) | (b2) | (c1) | (c2) | | | |
| Example | 12 | 100 | 0.8 | - | 0.3 | - | - | - | Addition of solution | 280 | 8 |
| | 13 | 100 | - | 0.8 | 0.3 | - | - | - | Addition of solution | 280 | 17 |
| | 14 | 100 | 0.8 | - | - | 0.3 | - | - | Addition of solution | 280 | 13 |
| | 15 | 100 | 0.3 | - | 0.3 | - | - | - | Addition of solution | 280 | 15 |
| | 16 | 100 | 2.0 | - | 0.3 | - | - | - | Addition of solution | 280 | 17 |
| | 17 | 100 | 0.8 | - | 1.0 | - | - | - | Addition of solution | 280 | 17 |
| | 18 | 100 | 0.8 | - | 0.3 | - | 0.05 | - | Addition of solution | 280 | 17 |
| | 19 | 100 | 0.8 | - | 0.3 | - | - | 0.05 | Addition of solution | 280 | 20 |
| | 20 | 100 | 0.1 | - | 0.1 | - | - | - | Addition of solution | 280 | 18 |
| | 21 | 100 | 2.0 | - | 1.0 | - | - | - | Addition of solution | 280 | 19 |
| | 22 | 100 | 0.8 | - | 0.3 | - | - | - | Twin-screw kneading | 280 | 15 |
| | 23 | 100 | 0.1 | - | 0.005 | - | - | - | Addition of solution | 280 | 18 |

(continued)

| | | Resin composition | | | | | | | | Forming condition | Thermal yellowing resistance test |
|---|---|---|---|---|---|---|---|---|---|---|---|
| | | Content (parts) | | | | | | | Method for adding additives | Cylinder temperature [°C] | $\Delta\delta$YI |
| | | | Hindered-phenol-based anti-oxidait | | Hindered amine compound | | Heteroatom-cortaining compound | | | | |
| | | Alicyclic- structure-containing polymer (Production Example 1) | (a1) | (a2) | (b1) | (b2) | (c1) | (c2) | | | |
| Comparative Example | 7 | 100 | 0.8 | - | 0.3 | - | 0.1 | - | Addition of solution | 280 | 48 |
| | 8 | 100 | 0.8 | - | 0.3 | - | - | 0.1 | Addition of solution | 280 | 55 |
| | 9 | 100 | 0.8 | - | - | - | - | - | Addition of solution | 280 | 25 |
| | 10 | 100 | 0.8 | - | 2.0 | - | - | - | Addition of solution | 280 | 30 |
| | 11 | 100 | 0.05 | - | 0.5 | - | - | - | Addition of solution | 280 | 40 |
| | 12 | 100 | 3.0 | - | 0.3 | - | - | - | Addition of solution | 280 | 23 |
| | 13 | 100 | 0.3 | - | 0.3 | - | - | - | Addition of solution | 320 | 25 |
| | 14 | 100 | 0.1 | - | 0.1 | - | - | - | Twin-screw kneading | 280 | 25 |
| | 15 | 100 | 0.3 | - | 0.002 | - | - | - | Addition of solution | 280 | 40 |
| | 16 | 100 | 0.8 | - | 0.002 | - | - | - | Addition of solution | 280 | 23 |

The followings can be seen from Table 3.

The resin formed articles 27 to 38 in Examples 27 to 38 are excellent in thermal yellowing resistance because every yellowness index ΔδYI after the thermal resistance test is 20 or lower.

On the other hand, both of the resin formed articles 39 and 40 in Comparative Examples 7 and 8 are poor in thermal yellowing resistance because they contain a large blending amount of heteroatom-containing compounds.

The resin formed articles 41, 42, 47 and 48 in Comparative Examples 9, 10, 15, and 16 are poor in thermal yellowing resistance because the blending amount of the hindered amine compound is out of an appropriate range.

All of the resin formed articles 43 and 44 in Comparative Examples 11 and 12 are poor in thermal yellowing resistance because the blending amount of the hindered-phenol-based antioxidant is out of an appropriate range.

The resin formed article 45 in Comparative Example 13 is poor in thermal yellowing resistance because the blending amount of the additives has been slightly small and the melting temperature during injection forming has been too high.

The resin formed article 46 in Comparative Example 14 is poor in thermal yellowing resistance because the blending amount of the additives has been slightly small and the twin-screw kneading has been adopted as a method for preparing the resin composition.

## Claims

1. A resin composition comprising an alicyclic-structure-containing polymer, a hindered-phenol-based antioxidant and a hindered amine compound, wherein

the hindered-phenol-based antioxidant is a compound having a group represented by the following formula (1):

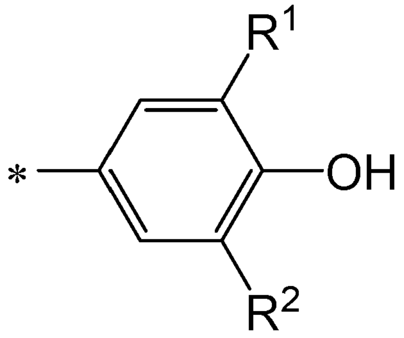

(1)

(wherein each of $R^1$ and $R^2$ independently represents a group having 1 or more carbon atoms, and the total number of carbon atoms constituting $R^1$ and $R^2$ is 2 to 20, and * represents a bonding hand.),
the hindered amine compound is a compound represented by the following formula (2a):

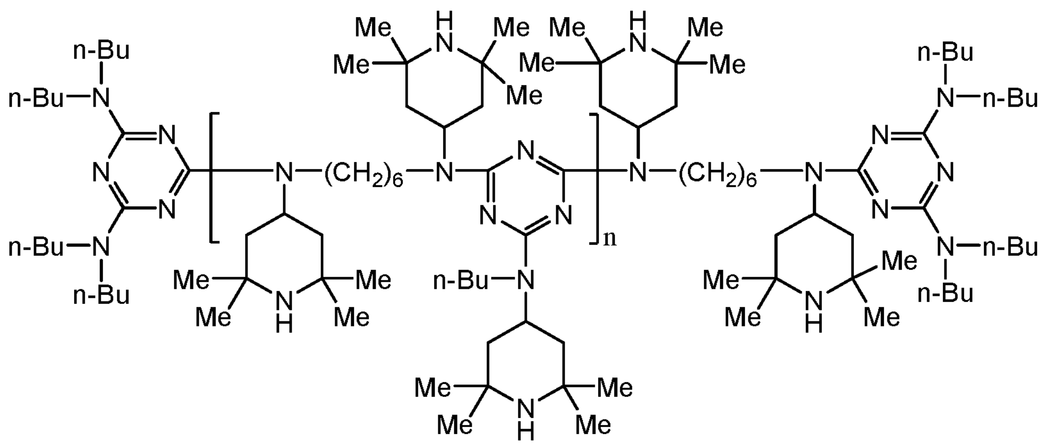

(2a)

wherein n represents any natural number, Me represents a methyl group, and n-Bu represents a n-butyl group,
a content of the hindered-phenol-based antioxidant is 0.2 to 2.0 parts by weight based on 100 parts by weight of the alicyclic-structure-containing polymer, and

a content of the hindered amine compound is 0.01 to 0.10 part by weight based on 100 parts by weight of the alicyclic-structure-containing polymer.

**2.** The resin composition according to claim 1, wherein

the content of the hindered-phenol-based antioxidant is 0.2 to 1.0 part by weight based on 100 parts by weight of the alicyclic-structure-containing polymer, and
the content of the hindered amine compound is 0.01 to 0.10 part by weight based on 100 parts by weight of the alicyclic-structure-containing polymer.

**3.** The resin composition according to claim 1 or 2, wherein the alicyclic-structure-containing polymer is a norbornene-based polymer.

**4.** A resin formed article produced by forming the resin composition according to any of claims 1 to 3.

**5.** The resin formed article according to claim 4, wherein a yellowness index (ΔδYI), measured in a transmission mode using a color difference meter in accordance with JIS K7373, represented by the following equation (I) is 20 or less when a test piece with an optical path length of 3 mm is prepared using the resin formed article as a material and the resulting test piece is allowed to stand under a condition of an oxygen concentration of 21 vol% and a temperature of 125°C for 1,000 hours to carry out a heat resistance test:

$$\Delta \delta YI = \delta YI_1 - \delta YI_0 \qquad (I)$$

wherein $\delta YI_1$ represents a difference between a yellowness index of the test piece after the heat resistance test ($YI_1$) and a yellowness index of a blank (air) ($YI_B$), and $\delta YI_0$ represents a difference between a yellowness index of the test piece before the heat resistance test ($YI_0$) and the yellowness index of the blank (air) ($YI_B$).

**6.** An optical member comprising the resin formed article according to claim 4 or 5.

**Patentansprüche**

**1.** Harzzusammensetzung umfassend ein alicyclische Strukturen enthaltendes Polymer, ein Antioxidationsmittel auf Basis von gehindertem Phenol und eine gehinderte Aminverbindung, wobei

das Antioxidationsmittel auf Basis von gehindertem Phenol eine Verbindung mit einer Gruppe der folgenden Formel (1) ist:

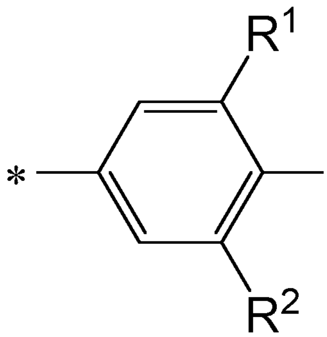

(1)

(worin $R^1$ und $R^2$ jeweils unabhängig voneinander eine Gruppe mit 1 oder mehreren Kohlenstoffatomen darstellen und die Gesamtzahl der Kohlenstoffatome, die $R^1$ und $R^2$ bilden, 2 bis 20 beträgt und * eine Bindungshand darstellt),
die gehinderte Aminverbindung eine Verbindung ist, die durch die folgende Formel (2a) dargestellt wird:

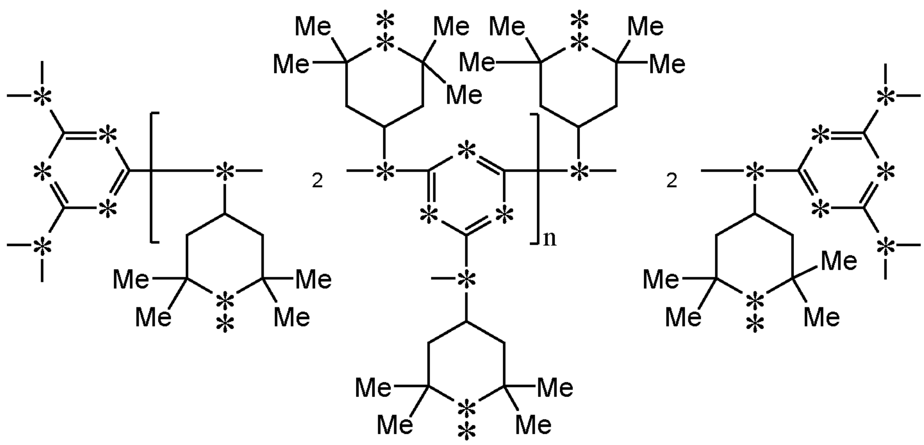

worin n eine beliebige natürliche Zahl, Me eine Methylgruppe und n-Bu eine n-Butylgruppe darstellen, der Gehalt des Antioxidationsmittels auf Basis von gehindertem Phenol 0,2 bis 2,0 Gewichtsteile, bezogen auf 100 Gewichtsteile des alicyclische Strukturen enthaltenden Polymers, beträgt und der Gehalt der gehinderten Aminverbindung 0,01 bis 0,10 Gew.-Teile, bezogen auf 100 Gew.-Teile des alicyclische Strukturen enthaltenden Polymers, beträgt.

**2.** Harzzusammensetzung nach Anspruch 1, wobei

der Gehalt des Antioxidationsmittels auf Basis von gehindertem Phenol 0,2 bis 1,0 Gewichtsteile, bezogen auf 100 Gewichtsteile des alicyclische Strukturen enthaltenden Polymers, beträgt, und der Gehalt an der gehinderten Aminverbindung 0,01 bis 0,10 Gewichtsteile, bezogen auf 100 Gewichtsteile des alicyclische Strukturen enthaltenden Polymers, beträgt.

**3.** Harzzusammensetzung nach Anspruch 1 oder 2, wobei das alicyclische Strukturen enthaltende Polymer ein Polymer auf Norbornenbasis ist.

**4.** Harzformgegenstand, hergestellt durch Formen der Harzzusammensetzung nach einem der Ansprüche 1 bis 3.

**5.** Harzformgegenstand nach Anspruch 4, wobei ein Vergilbungsindex ($\Delta\delta YI$), der in einem Transmissionsmodus unter Verwendung eines Farbdifferenzmessgeräts gemäß JIS K7373 gemessen wird und durch die folgende Gleichung (I) dargestellt wird, 20 oder weniger beträgt, wenn ein Teststück mit einer optischen Weglänge von 3 mm unter Verwendung des Harzformgegenstands als Material hergestellt wird und das resultierende Teststück unter einer Bedingung einer Sauerstoffkonzentration von 21 Vol.-% und einer Temperatur von 125°C 1.000 Stunden lang stehen gelassen wird, um einen Wärmebeständigkeitstest durchzuführen:

$$\Delta\delta YI = \delta YI_1 - \delta YI_0 \qquad (I)$$

wobei $\delta YI_1$ eine Differenz zwischen einem Vergilbungsindex des Teststücks nach dem Wärmewiderstandstest ($YI_1$ ) und einem Vergilbungsindex eines Rohlings (Luft) ($YI_B$ ) darstellt, und $\delta YI_0$ eine Differenz zwischen einem Vergilbungsindex des Teststücks vor dem Wärmewiderstandstest ($YI_0$ ) und dem Vergilbungsindex des Rohlings (Luft) ($YI_B$ ) darstellt.

**6.** Optisches Element, das den Harzformgegenstand nach Anspruch 4 oder 5 umfasst.

## Revendications

**1.** Composition de résine comprenant un polymère contenant une structure alicyclique, un antioxydant à base de phénol encombré et un composé de type amine encombrée, dans laquelle

l'antioxydant à base de phénol encombré est un composé ayant un groupe représenté par la formule (1) suivante :

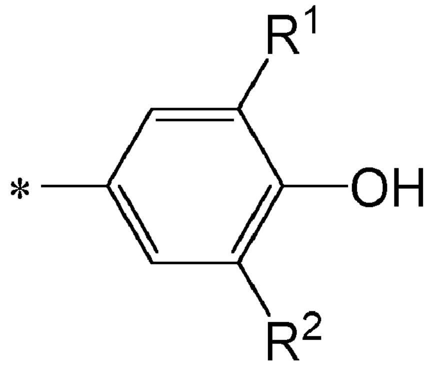

(1)

(dans laquelle chacun de $R^1$ et $R^2$ représente indépendamment un groupe ayant 1 ou plusieurs atomes de carbone, et le nombre total d'atomes de carbone constituant $R^1$ et $R^2$ est de 2 à 20, et * représente une extrémité de liaison),

le composé de type amine encombrée est un composé représenté par la formule (2a) suivante :

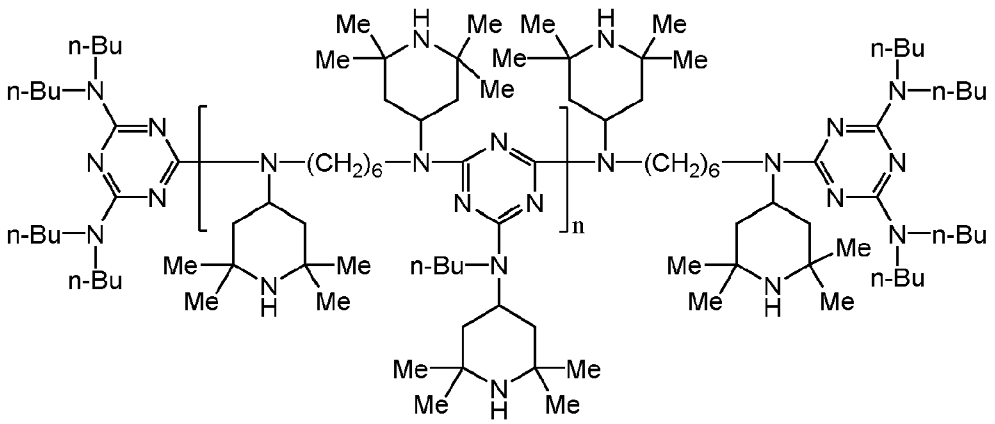

(2a)

dans laquelle n représente n'importe quel nombre entier naturel, Me représente un groupe méthyle, et n-Bu représente un groupe n-butyle,

la teneur en l'antioxydant à base de phénol encombré est de 0,2 à 2,0 parties en poids pour 100 parties en poids du polymère contenant une structure alicyclique, et

la teneur en le composé de type amine encombrée est de 0,01 à 0,10 partie en poids pour 100 parties en poids du polymère contenant une structure alicyclique.

**2.** Composition de résine selon la revendication 1, dans laquelle

la teneur en l'antioxydant à base de phénol encombré est de 0,2 à 1,0 partie en poids pour 100 parties en poids du polymère contenant une structure alicyclique, et

la teneur en le composé de type amine encombrée est de 0,01 à 0,10 partie en poids pour 100 parties en poids du polymère contenant une structure alicyclique.

**3.** Composition de résine selon la revendication 1 ou 2, dans laquelle le polymère contenant une structure alicyclique est un polymère à base de norbornène.

**4.** Article formé de résine produit par formation de la composition de résine de l'une quelconque des revendications 1 à 3.

**5.** Article formé de résine selon la revendication 4, dans lequel l'indice de jaunissement (DdYI), mesuré en mode transmission utilisant un dispositif de mesure de différence de couleur conformément à la norme JIS K7373, représenté par l'équation (I) qui suit, est de 20 ou moins quand une éprouvette ayant une longueur de trajet optique de 3 mm est préparée par utilisation de l'article formé de résine en tant que matériau et l'éprouvette résultante est laissée à reposer dans des conditions de concentration d'oxygène de 21 % en volume et de température de 125°C pendant 1000 heures pour la mise en oeuvre d'un test de résistance à la chaleur :

$$DdYI = dYI_1 - dYI_0 \qquad (I)$$

dans laquelle $dYI_1$ représente la différence entre l'indice de jaunissement de l'éprouvette après le test de résistance à la chaleur ($YI_1$) et l'indice de jaunissement d'un témoin à blanc (air) ($YI_B$), et $dYI_0$ représente la différence entre l'indice de jaunissement de l'éprouvette avant le test de résistance à la chaleur ($YI_0$) et l'indice de jaunissement d'un témoin à blanc (air) ($YI_B$).

**6.** Elément optique comprenant l'article formé de résine de la revendication 4 ou 5.

FIG. 1

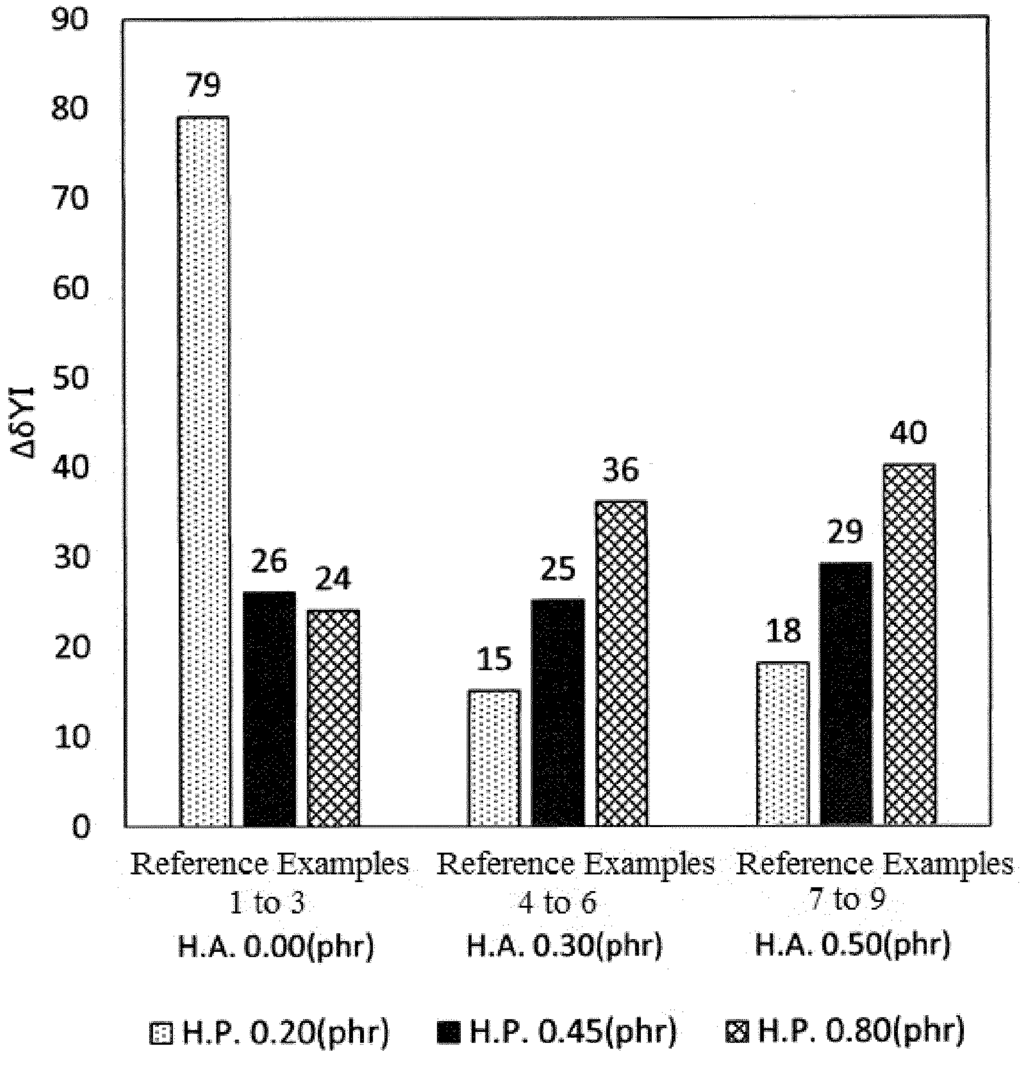

ΔδYI
90
80
70
60
50
40
30
20
10
0
79
26
24
15
25
36
18
29
40
Reference Examples 1 to 3
H.A. 0.00(phr)
Reference Examples 4 to 6
H.A. 0.30(phr)
Reference Examples 7 to 9
H.A. 0.50(phr)
H.P. 0.20(phr)
H.P. 0.45(phr)
H.P. 0.80(phr)

FIG. 2

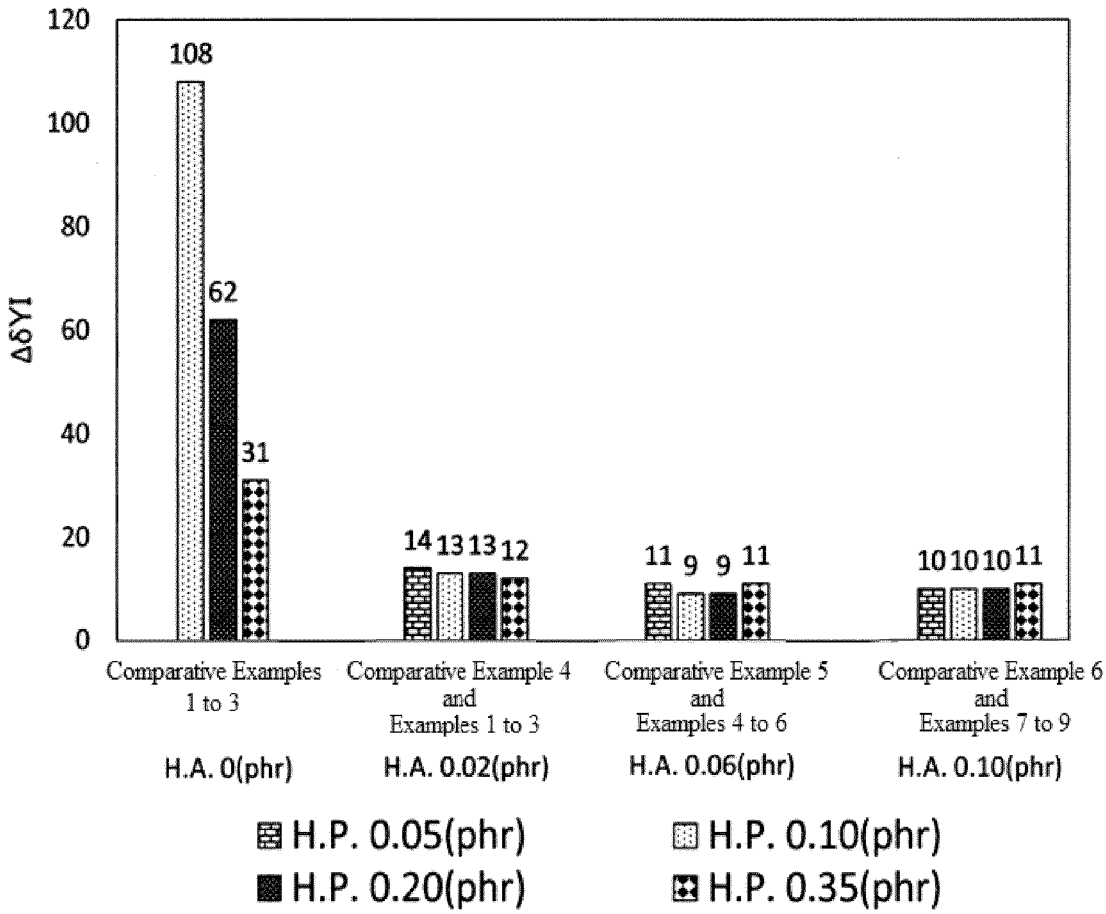

ΔδYI
120
100
80
60
40
20
0
108
62
31
14
13
13
12
11
9
9
11
10
10
10
11
Comparative Examples 1 to 3
Comparative Example 4 and Examples 1 to 3
Comparative Example 5 and Examples 4 to 6
Comparative Example 6 and Examples 7 to 9
H.A. 0(phr)
H.A. 0.02(phr)
H.A. 0.06(phr)
H.A. 0.10(phr)
H.P. 0.05(phr)
H.P. 0.10(phr)
H.P. 0.20(phr)
H.P. 0.35(phr)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012043721 A
- JP H09268259 A
- JP 2006143931 A
- JP 2007176960 A
- JP 2014065756 A
- WO 9938918 A1
- WO 2008047468 A1
- JP 2011052154 A